# FASCICULE DE BREVET EUROPEEN

(11) **EP 1 177 217 B1**
(45) Date de publication et mention de la délivrance du brevet: **13.10.2004**
(21) Numéro de dépôt: 00922751.3
(22) Date de dépôt: 26.04.2000
(51) Int. Cl.: C08B 37/16

(54) **CYCLODEXTRINES AMPHIPHILES, LEUR PREPARATION ET LEUR UTILISATION POUR SOLUBILISER DES SYSTEMES ORGANISES ET INCORPORER DES MOLECULES HYDROPHOBES**
AMPHIPHILE CYCLODEXTRINE, DEREN HERSTELLUNG UND VERWENDUNG ZUR AUFLÖSUNG VON STRUKTURIERTEN SYSTEMEN UND ZUR EINBETTUNG VON HYDROPHOBEN VERBINDUNGEN
AMPHIPHILE CYCLODEXTRINS, PREPARATION AND USE THEREOF FOR SOLUBILISING ORGANISED SYSTEMS AND INCORPORATING HYDROPHOBIC MOLECULES

(30) Priorité: 29.04.1999 FR 9905460
(43) Date de publication de la demande: 06.02.2002
(73) Titulaire: COMMISSARIAT A L'ENERGIE ATOMIQUE, 75752 Paris Cédex 15 (FR)
(72) Inventeur: AUZELY-VELTY, Rachel, F-38800 le Pont de Claix (FR); PERLY, Bruno, F-78320 la Verriere (FR); DJEDAINI-PILARD, Florence, F-91150 Etampes (FR)
(74) Mandataire: Audier, Philippe André
(86) Numéro de dépôt international: PCT/FR2000/001102
(87) Numéro de publication internationale: WO 2000/066635

(56) Documents cités:
- EP-A- 0 751 150
- FR-A- 2 681 868

## Description

### Domaine technique

La présente invention a pour objet de nouveaux dérivés de cyclodextrines, utilisables en particulier pour l'incorporation en milieu aqueux de composés chimiques hydrophobes tels que des molécules pharmaceutiquement actives, des molécules à applications cosmétiques et des molécules utilisées comme agents de contraste pour l'imagerie médicale.

De façon plus précise, elle concerne des dérivés amphiphiles de cyclodextrines présentant des propriétés d'auto-organisation en milieu aqueux et étant susceptibles de s'incorporer dans des systèmes de tensioactifs organisés conduisant à la formation de systèmes mixtes.

Cette incorporation dans des systèmes de tensioactifs organisés tels que des petites vésicules de phospholipides, est destinée à permettre le transport de molécules hydrophobes incluses dans la cyclodextrine, par exemple d'un principe actif, en particulier par voie transmembranaire, par exemple transdermique.

### État de la technique antérieure

Les cyclodextrines ou cyclomaltooligosaccharides sont des composés d'origine naturelle formés par l'enchaînement de 6, 7 ou 8 unités glucose liées en α-1→4. De nombreux travaux ont montré que ces composés pouvaient former des complexes d'inclusion avec des molécules hydrophobes permettant ainsi leur solubilisation dans des milieux aqueux. De nombreuses applications ont été proposées pour tirer profit de ce phénomène, en particulier dans le domaine pharmaceutique, comme il est décrit par D. Duchêne, dans « Pharmaceutical Applications of Cyclodextrins », paru dans « Cyclodextrins and their industrial uses », Editions de Santé, Paris 1987, pp. 213-257 [1]. Des compositions pharmaceutiques utilisant des cyclodextrines ont déjà été commercialisées au Japon, en Italie et plus récemment en France, par exemple par Pierre Fabre Médicament pour le Brexin® qui est un complexe d'inclusion du Piroxicam dans la β-cyclodextrine.

Parmi les cyclodextrines utilisables, la β-cyclodextrine (comportant 7 unités glucose), est la plus adaptée au niveau de la taille de sa cavité et la moins chère des trois. Des modifications chimiques de la β-cyclodextrine ont été décrites afin de la rendre amphiphile dans le but de l'incorporer à des systèmes organisés.

Ainsi, des cyclodextrines amphiphiles portant de multiples chaînes sur la face primaire ont été étudiées. A. Yabe et coll ont décrit dans « Thin Solid Films, (1988), 160, pp. 33-41 [2], le dérivé *per*(6-dodécylamino-6-désoxy)-β-cyclodextrine afin de former des couches de Langmuir-Blodgett stables. De même, L. Jullien et coll ont décrit dans J. Chem. Soc. Perkin Trans 2, 1993, pp. 1011-1022 [3], des dérivés de β-cyclodextrine comportant des chaînes aliphatiques situées en positions primaires et secondaires, en vue d'incorporer ces dérivés de cyclodextrines dans des vésicules de phosphatidylcholine. Ces dérivés sont amphiphiles et peuvent être incorporés dans les vésicules, mais la cavité interne de la cyclodextrine n'est plus accessible en raison du fort encombrement stérique des chaînes aliphatiques. Par conséquent, ces dérivés sont incapables d'inclure des molécules hydrophobes, en particulier des molécules de principe actif.

Récemment, il a été montré par A. Gulik et coll dans Langmuir (1998), 14, pp. 1050-1057 [4], que des cyclodextrines dites à « jupes », portant des chaînes d'acides gras greffées sur les hydroxyles secondaires pouvaient former des nanosphères stables. Ces super-assemblages moléculaires semblent présenter des propriétés d'encapsulation et de relargage très prometteuses en raison des effets cumulatifs de la spécificité de taille et de transport des cyclodextrines, d'une part, et d'organisation des nanoparticules, d'autre part. Toutefois, il faut souligner que la synthèse et surtout la purification de telles cyclodextrines restent très difficiles et nécessitent de longues étapes de purification conduisant à de faibles rendements. Il est évident que les propriétés d'organisation supramoléculaire sont drastiquement liées à la pureté chimique des dérivés de cyclodextrines amphiphiles.

J. Lin et coll ont décrit dans FR-A-2 736 056 [5] et dans J. Chem. Soc. Perkin Trans 2, (1998), pp. 2638-2646 [6], la synthèse de dérivés de cyclodextrines appelées « Bilboquets » comportant une ou plusieurs chaînes aliphatiques leur conférant des propriétés amphiphiles sans conduire pour autant au phénomène d'auto-inclusion de la ou des chaînes dans la cyclodextrine. De ce fait, on peut obtenir à partir de tels dérivés des complexes d'inclusion contenant une molécule hydrophobe et l'incorporation de ces complexes dans des vésicules phospholipidiques. Toutefois, ces molécules se sont révélées peu stables en milieu physiologique, soit à des pH égaux et. supérieurs à 7, et leurs capacités d'incorporation dans des systèmes organisés restent limitées. De plus, ces molécules Bilboquets ne s'auto-organisent pas spontanément en milieu aqueux pour donner des particules de taille et de forme bien définies.

Des dérivés amphiphiles de cyclodextrines obtenus par attachement de chaînes grasses aux groupes hydroxyles primaires ou secondaies des résidus glycosyles sont divulgués dans FR-A-2 681 868.

### Exposé de l'invention

La présente invention a précisément pour objet des dérivés amphiphiles de cyclodextrines, stables en milieu physiologique, susceptibles d'inclure des composés hydrophobes, ayant de bonnes capacités d'incorporation dans des systèmes organisés et présentant de plus des propriétés d'auto-organisation en milieu aqueux.

Selon l'invention, le dérivé amphiphile de cyclodextrine répondant à la formule : 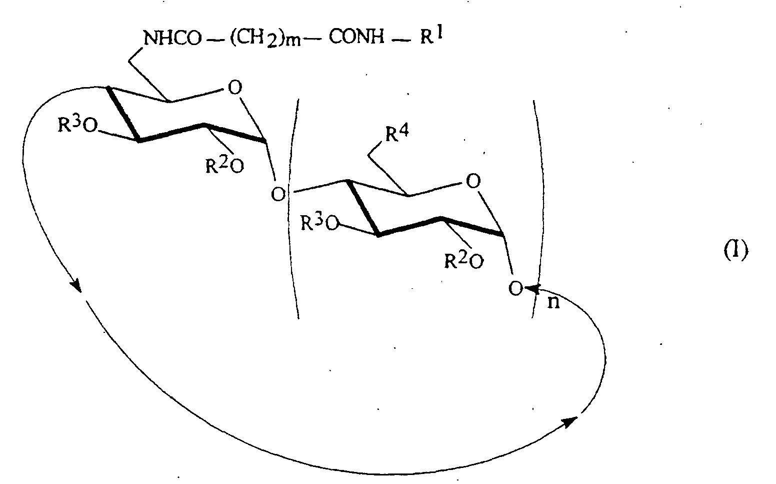 dans laquelle :
- R¹ représente un groupe dérivé d'un stéroïde,
- R² représente un groupe alkyle ou aryle éventuellement substitué,
- R³ représente H ou R²,
- tous les R⁴ représentent OR², ou
- l'un des R⁴ représente -NHCO(CH₂)ₘCONHR¹ et les autres R⁴ représentent OR² à condition qu'il y ait au moins une unité glucose avec R⁴ représentant OR² entre les deux unités glucose comportant le substituant ―NHCO―(CH₂)ₘ―CONH―R¹,
- m est un nombre entier allant de 1 à 8, et
- n est égal à 5, 6 ou 7.

On rappelle que les stéroïdes sont des composés dérivant d'un noyau polycyclique de formule : 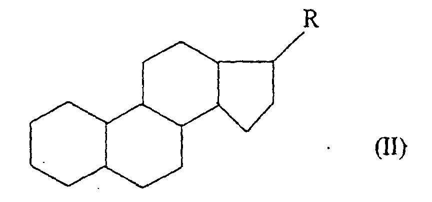 dans laquelle R représente un groupe hydrocarboné linéaire ou ramifié de 1 à 9 atomes de carbone, et
dans lesquels le noyau polycyclique peut comporter une ou plusieurs doubles liaisons, et un ou plusieurs substituants choisis parmi CH₃, OH et O, sur un ou plusieurs atomes de carbone des cycles.

Dans le dérivé de cyclodextrine de l'invention, R¹ peut représenter un groupe dérivé des stérols par élimination du groupe hydroxyle du premier cycle, ayant un degré d'insaturation de 0 à 6. Il peut s'agir aussi de groupes dérivés de stérones. Par exemple R¹ peut représenter un groupe dérivé du cholestérol tel que le groupe de formule :

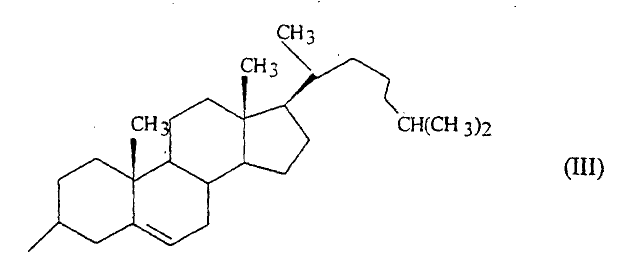

Dans le dérivé de l'invention, on obtient les propriétés amphiphiles grâce à la présence d'un ou deux substituants comportant un groupe dérivé d'un stéroïde.

Lorsque le dérivé comporte deux substituants de ce type, il est nécessaire qu'ils ne se trouvent pas sur deux unités glucose adjacentes de la cyclodextrine, en raison de leur encombrement.

Aussi, les deux unités glucose comportant ces substituants sont séparées par une ou deux unités glucose ayant un substituant OR².

De préférence, le dérivé de cyclodextrine ne comporte qu'un seul substituant de ce type, tous les R⁴ représentant OR².

Dans le dérivé de cyclodextrine de l'invention, le groupe R² représente un groupement alkyle linéaire ou ramifié ou aryle, éventuellement substitué. Lorsque l'on utilise un groupe alkyle, celui-ci a généralement de 1 à 4 atomes de carbone et il est de préférence linéaire. Le groupe aryle peut être par exemple le groupe phényle ou le groupe benzyle. Les substituants éventuels de ces groupes alkyle ou aryle peuvent être par exemple des atomes d'halogènes et des groupes hydroxyle, carboxyle et amine. Avantageusement, R² représente le groupe méthyle.

R³ peut représenter un atome d'hydrogène ou un groupe alkyle identique ou différent de R². De préférence, R³ représente H.

Dans la formule (I) donnée ci-dessus, la chaîne aliphatique reliant le groupe dérivé d'un stéroïde à l'unité glucose peut comporter entre les deux groupes amido, de 1 à 8 atomes de carbone. On obtient de bons résultats avec deux atomes de carbone, soit avec m égal à 2.

Les dérivés de cyclodextrine de l'invention peuvent être des dérivés de l'α-, β- ou γ-CD. De préférence, on utilise les dérivés de la β-CD ce qui correspond dans la formule (I) donnée ci-dessus au cas où n est égal à 6.

Les dérivés de cyclodextrine de l'invention peuvent être préparés par des procédés classiques à partir des dérivés mono-azido ou diazido de cyclodextrines correspondants.

Dans le cas où l'on veut préparer un dérivé de formule (I) tel que défini ci-dessus avec R³ représentant un atome d'hydrogène, le procédé comprend les étapes suivantes :
a) faire réagir un dérivé de formule : 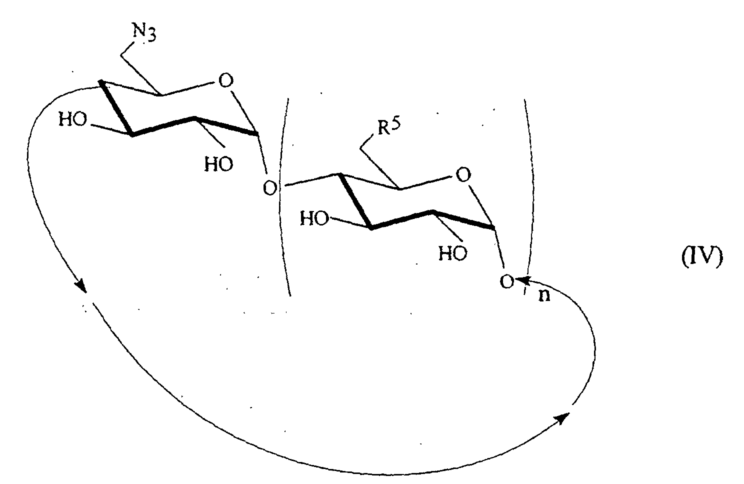 dans laquelle tous les R⁵ représentent OH, ou l'un des R⁵ représente ―N₃ et les autres R⁵ représentent OH à condition qu'il y ait au moins une unité glucose avec R⁵ représentant OH entre les deux unités glucose comportant le substituant N₃, et n est égal à 5, 6 ou 7,
   avec un sulfate de dialkyle SO₄R²₂ avec R² ayant la signification donnée ci-dessus, en milieu basique pour obtenir le dérivé de cyclodextrine de formule : 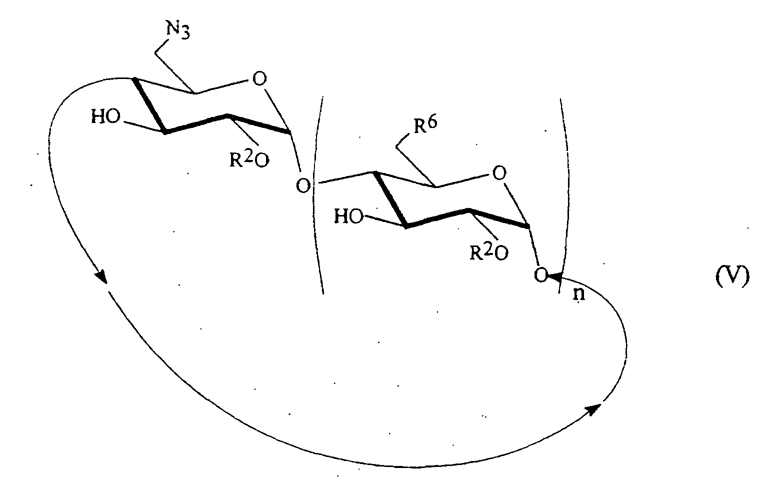 dans laquelle tous les R⁶ représentent OR², ou l'un des R⁶ représente N₃ et les autres R⁶ représentent OR², et R² et n sont tels que définis ci-dessus,
b) effectuer une réaction de Staudinger sur le dérivé de formule (V) à l'aide de triphénylphosphine et d'ammoniaque pour convertir N₃ en NH₂ et obtenir le dérivé de formule : 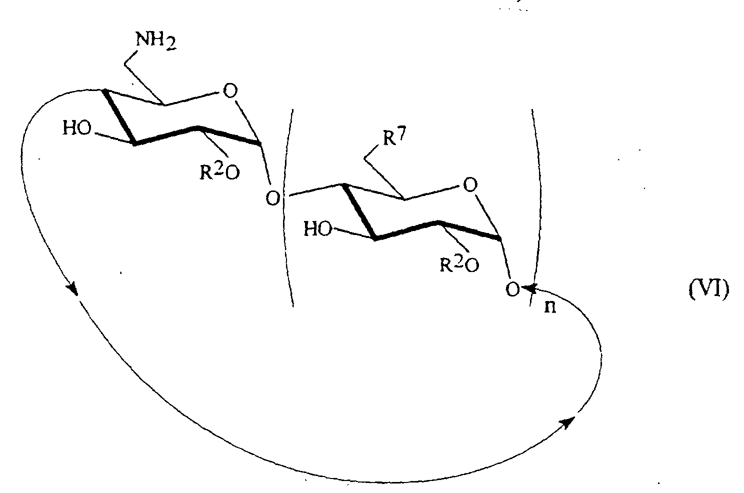 dans laquelle tous les R⁷ représentent OR², ou l'un des R⁷ représente NH₂ et les autres R⁷ représentent OR², et R² et n sont tels que définis ci-dessus,
c) faire réagir le dérivé de formule (VI) avec un anhydride d'acide de formule : 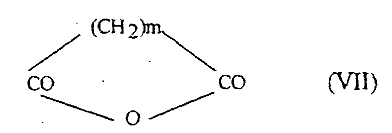 où m est tel que défini ci-dessus, pour obtenir le dérivé de formule : 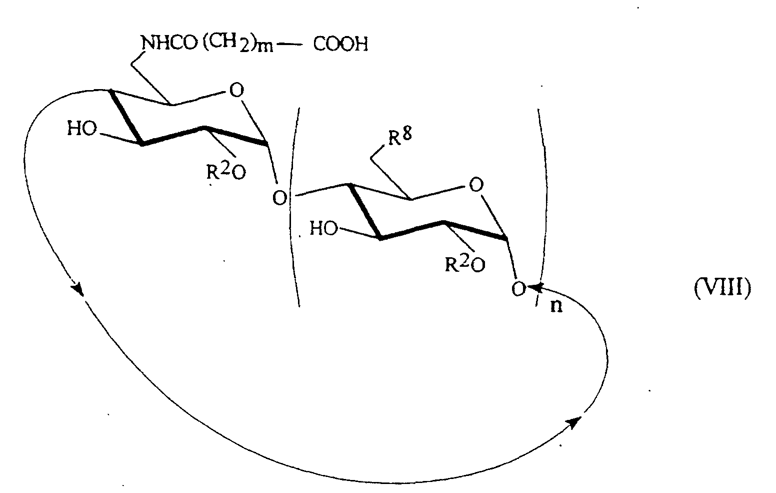 dans laquelle tous les R⁸ représentent OR² ou l'un des R⁸ représente ―NHCO―(CH₂)ₘ―COOH et les autres R⁸ représentent OR², et R², m et n sont tels que définis ci-dessus, et
d) faire réagir le dérivé de formule (VIII) avec un composé de formule NH₂-R¹ pour obtenir le dérivé de cyclodextrine de formule (I) défini ci-dessus.

Les dérivés monoazido ou diazido utilisés comme produit de départ dans le procédé peuvent être obtenus à partir du dérivé de cyclodextrine correspondant monotosylé ou ditosylé par action d'azoture de lithium dans l'eau.

Dans l'étape a) du procédé décrit ci-dessus, on fait réagir le dérivé de cyclodextrine de formule (IV) avec un sulfate de dialkyle SO₄R²₂ dans un mélange de solvants organiques tels que le dimethylformamide (DMF) et le diméthylsulfoxide (DMSO) dans des proportions 50:50 en volume, en présence d'une base telle que l'oxyde de baryum et l'hydroxyde de baryum, à 8°C. On peut séparer le dérivé de formule (V) ainsi obtenu en utilisant les procédés décrits en détails dans l'exemple 1.

Dans l'étape b), on fait réagir le dérivé de formule (V) avec de la triphénylphosphine dans un solvant organique tel que le DMF puis on additionne de l'ammoniaque à 20%. Le dérivé de formule (VI) ainsi obtenu peut être purifié par évaporation du solvant, élimination par filtration du précipité blanc formé puis séparation par chromatographie échangeuse d'ions. Dans l'étape c), on fait réagir le dérivé de formule (VI) avec l'anhydride d'acide de formule (VII) voulu dans un solvant organique tel que le DMF. Le dérivé de formule (VIII) obtenu n'est pas isolé et l'étape d) suivante se fait directement dans le même milieu réactionnel. On additionne alors des réactifs de couplage peptidique tels que le *N,N'*-diisopropylcarbodiimide et l'hydroxybenzotriazole. Le dérivé de formule (VIII) réagit alors avec le composé de formule H₂N-R¹ tel que la cholest-5-èn-3α-ylamine. On peut séparer le dérivé de formule I ainsi obtenu du milieu réactionnel par évaporation du solvant et purification par chromatographie sur colonne de gel de silice.

Dans le cas où l'on veut préparer un dérivé de formule (I) tel que défini ci-dessus, avec R³ représentant R², le procédé comprend les mêmes étapes que ci-dessus, mais dans l'étape a), on réalise une alkylation de tous les groupes OH par un iodoalcane. Dans ce cas, on réalise les étapes suivantes :
a) faire réagir un dérivé de formule : 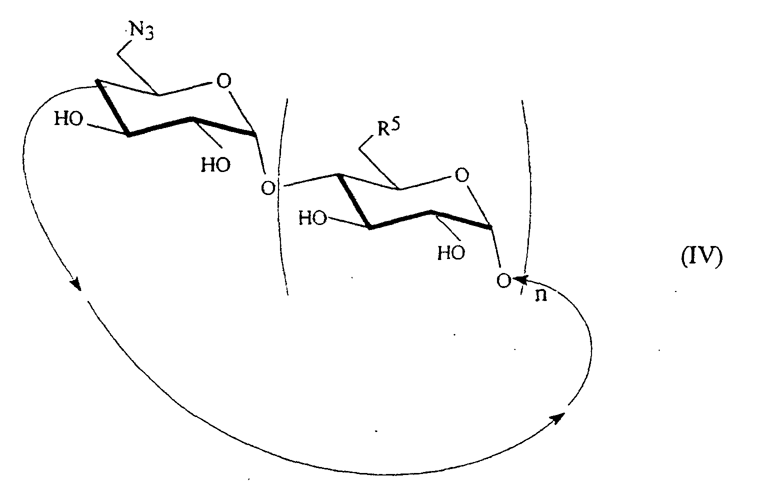 dans laquelle tous les R⁵ représentent OH, ou l'un des R⁵ représente ―N₃ et les autres R⁵ représentent OH à condition qu'il y ait au moins une unité glucose, avec R⁵ représentant OH entre les deux unités glucose comportant le substituant N₃, et n est égal à 5, 6 ou 7,
   avec un iodoalcane de formule IR² dans laquelle R² a la signification donnée ci-dessus, en présence de NaH pour pour obtenir le dérivé de cyclodextrine de formule : 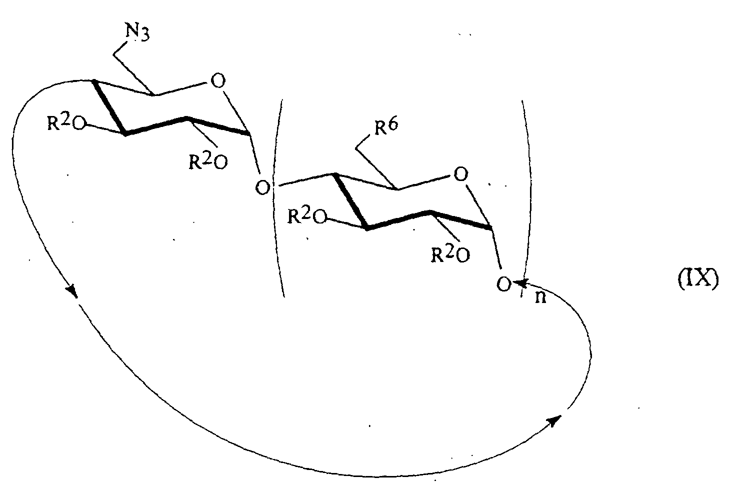 dans laquelle tous les R⁶ représentent OR², ou l'un des R⁶ représente N₃ et les autres R⁶ représentent OR², et R² et n sont tels que définis ci-dessus,
b) effectuer une réaction de Staudinger sur le dérivé de formule (IX) à l'aide de triphénylphosphine et d'ammoniaque pour convertir N₃ en NH₂ et obtenir le dérivé de formule : 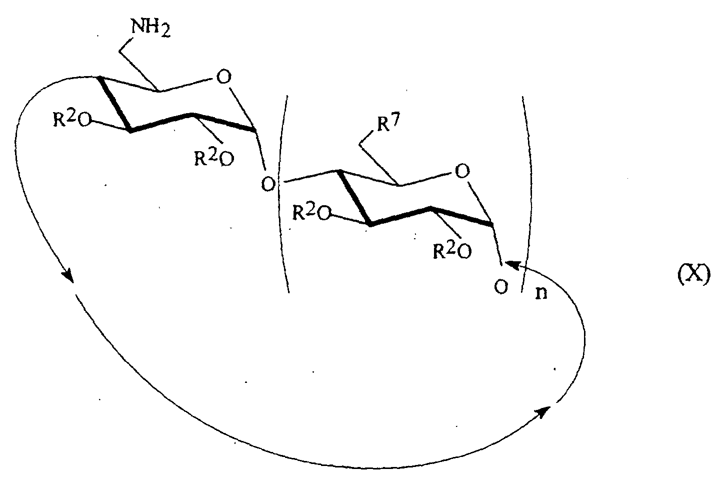 dans laquelle tous les R⁷ représentent OR², ou l'un des R⁷ représente NH₂ et les autres R⁷ représentent OR², et R² et n sont tels que définis ci-dessus,
c) faire réagir le dérivé de formule (X) avec un anhydride d'acide de formule : 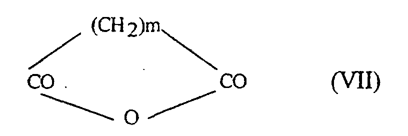 où m est tel que défini ci-dessus, pour obtenir le dérivé de formule : 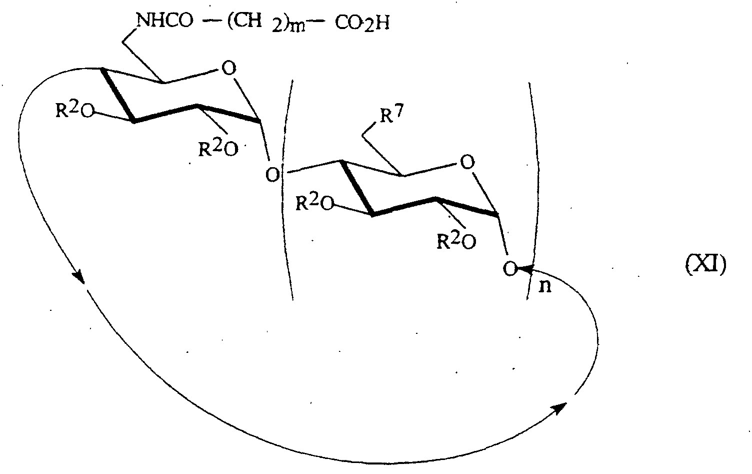 dans laquelle tous les R⁷ représentent OR² ou l'un des R⁷ représente ―NHCO―(CH₂)ₘ―COOH et les autres R⁷ représentent OR², et R², m et n sont tels que définis ci-dessus, et
d) faire réagir le dérivé de formule (XI) avec un composé de formule NH₂-R¹ pour obtenir le dérivé de cyclodextrine de formule (I) défini ci-dessus.

L'invention a encore pour objet les complexes d'inclusion du dérivé de cyclodextrine de formule (I) avec un composé hydrophobe en vue de solubiliser ce composé hydrophobe dans un milieu aqueux. Les composés chimiques hydrophobes susceptibles d'être solubilisés dans des milieux aqueux au moyen de ces dérivés de cyclodextrine (I) peuvent être de différents types. A titre d'exemple de tels composés, on peut citer des produits cosmétiques, des vitamines, des molécules pharmaceutiquement actives et des molécules utilisées comme agents de contraste pour l'imagerie médicale, par exemple, les composés décrits par Uekama et Irie dans Chemical Review (1998), 98, pp. 2045-2076 [7].

De préférence dans l'invention, le composé chimique hydrophobe est une molécule pharmaceutiquement active. A titre d'exemples de telles molécules, on peut citer les stéroïdes, par exemple la prednisolone, les neurotropes comme la dothiépine, les bactériostatiques comme le chloramphénicol, les vitamines comme la vitamine A, des toniques de la paroi vasculaire comme l'esculine, et des agents de contraste pour l'imagerie médicale comme l'acide 16-iodo-3-méthylhexadécanoïque.

Ces complexes d'inclusion peuvent être préparés par des procédés classiques, par exemple en ajoutant à une solution ou à une suspension de la cyclodextrine de formule (I) utilisée, une solution du composé hydrophobe dans un solvant organique approprié, par exemple l'acétone.

Les dérivés de cyclodextrine de formule (I) ont la propriété de s'auto-organiser spontanément en milieu aqueux pour donner des nanoparticules de 25 à 30 Å de rayon moyen et de forme parfaitement sphérique. Le nombre moyen de monomères est de 24 molécules de dérivés de cyclodextrine par nanoparticule. Aussi l'invention a également pour objet une solution aqueuse de nanoparticules d'un dérivé de cyclodextrine de formule (I) seul ou sous forme de complexe d'inclusion avec un composé hydrophobe.

Cette solution de nanoparticules peut être préparée en formant une solution aqueuse du dérivé de cyclodextrine ou d'un complexe d'inclusion de ce dérivé ayant une concentration en dérivé ou complexe supérieure à la concentration micellaire critique du dérivé.

L'auto-organisation des cyclodextrines amphiphiles en nanoparticules dans un milieu aqueux, permet d'assurer le transport d'une molécule hydrophobe, par exemple un principe actif, en particulier par voie transmembranaire ou parentérale.

Les dérivés de cyclodextrines de l'invention sont de plus particulièrement intéressants car ils peuvent être incorporés dans des systèmes organisés de tensioactifs tels que des petites vésicules de phospholipide ou des micelles. Cette incorporation est destinée à permettre la solubilisation de systèmes organisés, en vue d'assurer le transport de principes actifs inclus dans le dérivé de cyclodextrine.

Aussi, l'invention a aussi pour objet un système de tensioactifs organisé comprenant un dérivé de cyclodextrine ou un complexe d'inclusion de ce dérivé conformes à l'invention. Les tensioactifs susceptibles de former de tels systèmes organisés peuvent être de différents types. A titre d'exemple, on peut citer les phospholipides répondant à la formule générale suivante : 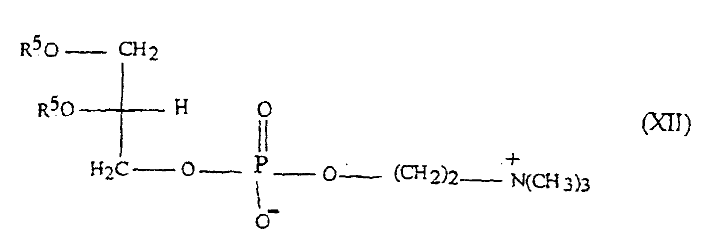 dans laquelle R⁵ représente CH₃-(CH₂)ₚ-CO avec p étant un nombre entier allant de 6 à 18. Ces phospholipides sont capables de former dé petites vésicules unilamellaires. C'est le cas en particulier de la dimyristoylphosphatidylcholine (DMPC) qui répond à la formule ci-dessus avec p = 12.

Pour incorporer le dérivé de cyclodextrine ou un complexe d'inclusion de ce dérivé conformes à l'invention dans le système organisé de tensioactifs, on peut préalablement former des petites vésicules de DMPC par sonication puis ajouter dans la solution aqueuse le dérivé de cyclodextrine ou le complexe d'inclusion. Le système mixte ainsi obtenu devient alors parfaitement soluble dans l'eau conduisant à une solution limpide. Le système mixte obtenu dans ce cas précis est une micelle mixte d'un rayon moyen de 60 Å.

Aussi l'invention a aussi pour objet une solution aqueuse comprenant en solution un système mixte formé à partir de vésicules de phospholipides ou de protéines membranaires, et d'au moins un dérivé de cyclodextrine ou d'au moins un complexe d'inclusion de dérivé de cyclodextrine conformes à l'invention.

De telles solutions sont intéressantes car elles permettent d'assurer le transport de molécules hydrophobes, par exemple d'un principe actif par voie transmembranaire ou parentérale, pour des applications pharmaceutiques et cosmétiques.

D'autres caractéristiques et avantages de l'invention apparaîtront mieux à la lecture des exemples suivants donnés bien entendu à titre illustratif et non limitatif, en référence aux figures 1 à 5 annexées.

### Brève description des dessins

La figure 1 illustre le spectre de diffusion de neutrons expérimental, en échelle logarithmique, d'une solution aqueuse de nanoparticules du dérivé de cyclodextrine obtenu dans l'exemple 1 avec trois courbes théoriques de diffusion de micelles sphériques, de micelles cylindriques et de bicouches.

La figure 2 illustre l'aspect de différents mélanges de cyclodextrine et de phospholipide.

Les figures 3, 4 et 5 illustrent respectivement les spectres de résonance magnétique nucléaire du ³¹P obtenus à partir de l'échantillon a (figure 3), b (figure 5) et d (figure 4) de l'exemple 3.

La figure 6 illustre le spectre de diffusion de neutrons expérimental, en échelle logarithmique, d'un mélange DMPC/dérivé de cyclodextrine (échantillon d) obtenu dans l'exemple 3 avec trois courbes théoriques de diffusion de micelles sphériques, de micelles cylindriques et de bicouches.

La figure 7 illustre les spectres de diffusion de neutrons expérimentaux en échelle logarithmique de mélanges d'acide 16-iodo-3-méthylhexadécanoïque et du dérivé de cyclodextrine de l'exemple 1 (1/1 et 0,5/1 mol) ainsi que le spectre de diffusion de neutron du dérivé de cyclodextrine de l'exemple 1 seul avec la courbe de diffusion théorique de nanoparticules seules.

### Exposé détaillé des modes de réalisation.

### Exemple 1: Synthèse du mono-6-(cholest-5-èn-3α-ylamido)succinylamido-6-désoxy-2,2',2'',2''',2'''', 2''''',2''''', 6',6'',6''',6'''',6''''',6''''''-tridéca-O-méthyl-cyclomaltoheptaose.

Ce composé est le dérivé de formule (I) avec R¹ représentant le groupe de formule (III), R² étant le groupe méthyle, R³ représentant H, tous les R⁴ représentant OCH₃, m étant égal à 2 et n étant égal à 6.
a) Préparation du mono-6-azido-6-désoxy-2,2',2'',2''',2'''',2''''',2'''''',6',6'',6''',6'''', 6''''',6''''''-tridéca-O-méthyl-cyclomaltoheptaose.
   Dans un ballon, on dissout 2 g (1,7 mmol) de mono-6-azido-6-desoxy-cyclomaltoheptaose (obtenu par exemple selon le protocole décrit dans Tetrahedron Lett. (1993), 34, pp. 2457-2460 [8] et J. Chem. Soc. Perkin Trans 2 (1995), pp. 723-730) [9]) dans 15 mL de diméthylsulfoxide anhydre. Cette solution est additionnée de 15 mL de diméthylformamide (DMF) anhydre. On ajoute ensuite sous atmosphère d'azote et sous vive agitation, 3,8 g (∼12 mmol) d'hydroxyde de baryum octahydraté et 3,6 g (∼24 mmol) d'oxyde de baryum. Après homogénéisation du milieu, on ajoute 8 mL de sulfate de diméthyle (∼84 mmol), et on laisse sous vive agitation, sous atmosphère d'azote, pendant 30 heures à 8 °C. La suspension d'un aspect laiteux est ensuite additionnée de 5 mL d'ammoniaque à 20 % et agitée pendant 3 heures à température ambiante. On laisse décanter au réfrigérateur pendant une nuit. Après concentration du surnageant sous pression réduite, le solide résiduel est repris avec 100 mL de dichlorométhane, et encore deux fois avec 50 mL de dichlorométhane. Les phases organiques sont rassemblées., lavées 3 fois avec 20 mL d'une solution aqueuse saturée en chlorure de sodium, 2 fois avec 20 mL d'eau, séchées sur sulfate de magnésium et concentrées sous pression réduite. Le produit est précipité par addition de 100 mL de n-hexane, filtré, lavé avec 100 mL de n-hexane et séché sous vide.
   On recueille 0,80 g (0,60 mmol) de mono-6-azido-6-désoxy-2,2',2'',2''',2'''',2''''', 2'''''',6',6'',6''',6'''',6''''',6''''''-tridéca-O-méthyl-cyclomaltoheptaose qui se présente sous la forme d'une poudre blanche.
b) Préparation du mono-6-amino-6-désoxy-2, 2',2'',2''',2'''',2''''',2'''''',6',6'',6''',6'''', 6''''',6''''''-tridéca-O-méthyl-cyclomaltoheptaose.
   On dissout 0,75 g (0,56 mmol) du composé obtenu en a) dans 30 mL de DMF. A cette solution, on ajoute goutte à goutte et sous agitation à température ambiante 0,75 g (2,86 mmol) de triphénylphosphine dans 5 mL de DMF. Le milieu réactionnel est maintenu à température ambiante pendant 2 heures, refroidi à 0°C et traité par 14 mL d'ammoniaque à 20 %. On laisse 18 heures à température ambiante sous agitation, puis le solvant est éliminé sous pression réduite et le solide résiduel est repris avec 30 mL d'eau. L'abondant insoluble de triphénylphosphine et de l'oxyde correspondant est éliminé par filtration. La solution est concentrée sous vide et le produit est purifié par chromatographie sur colonne de résine échangeuse d'ions (résine Lewatit® SP 1080 sous forme H⁺). On recueille 0,35 g (0,27 mmol) de mono-6-amino-6-désoxy-2,2',2'',2''',2'''',2''''',2'''''',6',6'',6''',6'''', 6''''',6''''''-tridéca-O-méthyl-cyclomaltoheptaose qui se présente sous la forme d'une poudre blanche.
c) Préparation du mono-6-(cholest-5-èn-3α-ylamido)succinylamido-6-désoxy-2,2',2'',2''',2'''', 2''''',2'''''',6',6'',6''',6'''',6''''',6''''''-tridéca-O-méthyl-cyclomaltoheptaose.
   A une solution de 0,25 g (0,19 mmol) du composé obtenu en b) dans 6 mL de DMF anhydre, on ajoute sous atmosphère d'azote et sous agitation à température ambiante 0,019 g (0,19 mmol) d'anhydride succinique dans 2 mL de DMF anhydre. Le milieu réactionnel est maintenu à température ambiante pendant 5 heures, puis additionné de 0,11 mL (0,76 mmol) de N,N'-diisopropylcarbodiimide et de 0,028 g (0,19 mmol) d'hydroxybenzotriazole dans 2 mL de DMF anhydre. Après 30 minutes d'agitation à température ambiante, on ajoute 0,089 g (0,23 mmol) de cholest-5-èn-3α-ylamine (obtenu en deux étapes à partir du cholest-5-èn-3β-ol selon les protocoles décrits dans Tetrahedron Lett. (1977), pp. 1977-1980 [10]). Le mélange réactionnel est laissé sous agitation à température ambiante pendant 48 heures, hydrolysé par addition de 0,30 mL d'eau et concentré sous pression réduite. Le solide résiduel est purifié par chromatographie sur colonne de gel de silice (gel de silice 60 Fluka; éluant : CH₂Cl₂-MeOH 95:5 puis 9:1 (v/v)). On recueille 0,24 g de mono-6-(cholest-5-èn-3α-ylamido)succinylamido-6-désoxy-2,2', 2'',2''',2'''',2''''',2'''''',6',6'',6''',6'''',6''''', 6''''''-tridéca-O-méthyl-cyclomaltoheptaose.
   (71 % de rendement en composé pur final à partir du mono-6-amino-6-désoxy-2,2',2'',2''',2'''', 2''''',2'''''',6',6'',6''',6'''',6''''',6''''''-tridéca-O-méthyl-cyclomaltoheptaose).

Les caractéristiques de ce composé sont les suivantes :
- Chromatographie sur couches minces (Plaques de Silice Merck) Rf = 0,50 dans le mélange CH₂Cl₂-MeOH 9:1 (v/v), révélation par H₂SO₄ 10 %.
- Spectrométrie de masse: ESI-MS : m/z = 1805,95 [M+Na]⁺ pour C₈₆ H₁₄₆ N₂O₃₆Na.
- RMN ¹H (500 MHz, 25°C, solution 7 mM dans CDCl₃) : attribution par des expériences COSY et COSY relais : δ = 6,49 (NH CD), 5,70 (NH Chol), 5,38 (H-6 Chol), 5,28-4,88 (H-1, OH-3 CD), 4,11 (H-3 Chol), 3,99-3,16 (H-2, H-3, H-4, H-5, H-6, H-6', OCH₃ CD), 2,60-2,48 (CH₂ succ, H-4 Chol), 2,04-0,68 (H Chol).

### Exemple 2: Préparation de nanoparticules de mono-6-(cholest-5-èn-3α-ylamido)succinylamido-6-désoxy-2,2',2'',2''',2'''',2''''',2'''''',6',6'',6''',6'''',6' '''',6''''''-tridéca-O-méthyl-cyclomaltoheptaose.

On prépare des nanoparticules de mono-6-(cholest-5-èn-3α-ylamido)succinylamido-6-désoxy-2,2', 2'',2''',2'''',2''''',2'''''',6',6'',6''',6'''',6''''', 6''''''-tridéca-*O*-méthyl-cyclomaltoheptaose simplement en formant une solution aqueuse de cette cholestéryl-cyclodextrine à une concentration supérieure à sa concentration micellaire critique (cmc).

La cmc de la cyclodextrine de l'exemple 1 a été déterminée par des mesures de tension de surface. La valeur de la cmc est de 9.10⁻⁶ mol/L.

Le diamètre hydrodynamique moyen des nanoparticules a été mesuré par diffusion quasi-élastique de la lumière. La valeur du diamètre moyen (DM) calculée selon l'approximation de Stokes-Einstein basée sur le modèle des sphères parfaites sans interactions est de 0,6 nm (60 Å). L'analyse par diffusion statique de la lumière de solutions aqueuses de nanoparticules à différentes concentrations (2,5.10⁻³, 5.10⁻³ et 10⁻² mol/L) donne une masse moyenne des agrégats de 43000 g/mol, ce qui correspond à 24 monomères en moyenne par nanoparticule.

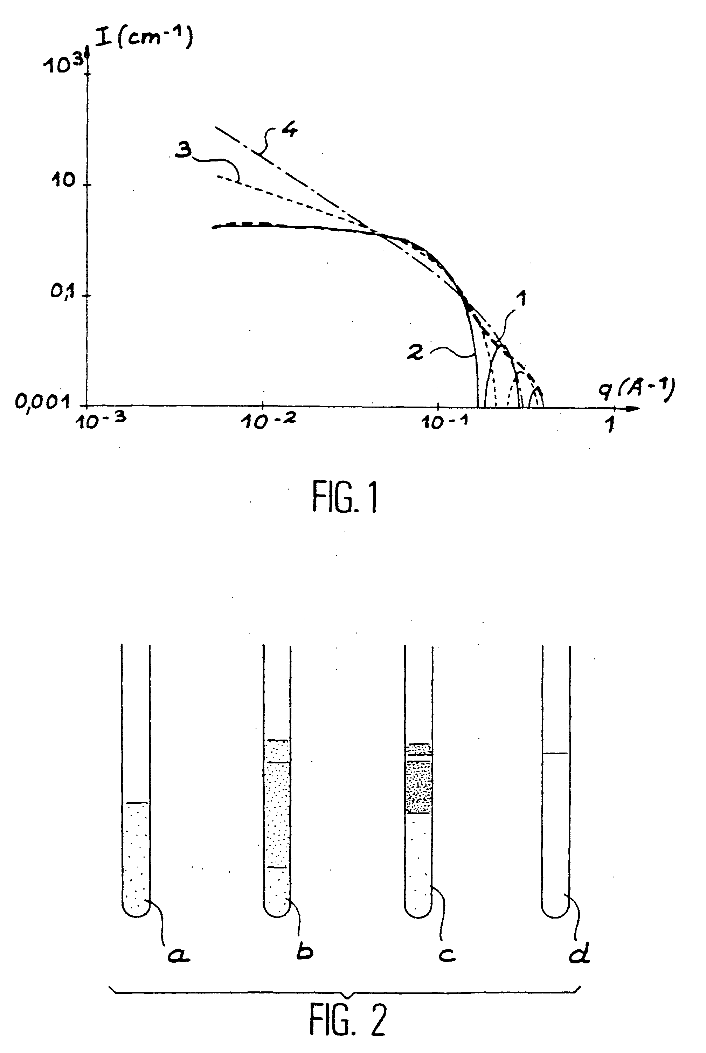

La forme parfaitement sphérique ainsi que la taille des nanoparticules ont été confirmées par diffusion de neutrons. Le spectre de diffusion obtenu à partir d'une solution 10⁻² mol/l dans D₂O du dérivé de cyclodextrine de l'exemple 1 est représenté sur la figure 1 (spectre 1).

Sur la figure 1, on a également représenté les spectres théoriques simulant des sphères (spectre 2), des cylindres (spectre 3) ou des lamelles (spectre 4) formés à partir du dérivé de cyclodextrine de l'exemple 1, à une concentration de 10⁻² mol/L dans D₂O. La superposition du spectre 2 simulant les sphères avec le spectre expérimental 1 prouve la formé sphérique des agrégats de cholestéryl-cyclodextrine. Ces agrégats sont tapissés à la surface de cavités de cyclodextrines disponibles pour l'inclusion de molécules actives hydrophobes, le coeur étant constitué des groupements cholestérol. Le spectre théorique simulant les sphères donne un diamètre moyen de 0,5 nm (50 Å) et un nombre moyen de monomères par nanoparticule de 24.

### Exemple 3: Préparation des systèmes mixtes de mono-6-(cholest-5-èn-3α-ylamido)succinylamido-6-désoxy-2,2', 2'',2''',2'''',2''''',2'''''',6',6'',6''',6'''',6''''', 6''''''-tridéca-O-méthyl-cyclomaltoheptaose et de Di-Myristoyl-Phosphatidyl-Choline (DMPC).

On prépare une suspension aqueuse à 15.10⁻³ mol/L de DMPC soit sous la forme de vésicules multilamellaires de grande taille (MLVs), soit sous la forme de vésicules unilamellaires de petite taille (SUVs), en suivant les protocoles de préparation décrits par exemple dans "Liposomes: a practical approach", R.R.C. New Ed., IRL Press, Oxford University Press, 1990 [11]. On ajoute à la suspension de MLVs ou de SUVs de DMPC la cyclodextrine de l'exemple 1 de façon à ce que la concentration finale de la cyclodextrine dans le mélange aqueux cyclodextrine/DMPC soit de 0,5.10⁻³ ou 2,5.10⁻³ mol/L.

La figure 2 illustre l'aspect des différents mélanges après 12 h à 25°C. Dans le tube a, il s'agit d'une suspension aqueuse de vésicules unilamellaires de DMPC 15 mM. Les tubes c et d correspondent aux mélanges DMPC/cyclodextrine : 15.10⁻³/0,5.10⁻³ et 15.10⁻³/2,5.10⁻³ mol/L respectivement. Le tube b est un tube "témoin" correspondant au mélange DMPC/heptakis(2,6-di-*O*-méthyl)cyclomaltoheptaose 15.10⁻³/2,5.10⁻³ mol/L, soit un mélange de DMPC avec une cyclodextrine ne comportant pas de substituant stéroïde.

Les différents mélanges sont examinés par spectroscopie RMN du ³¹P à 81 MHz.

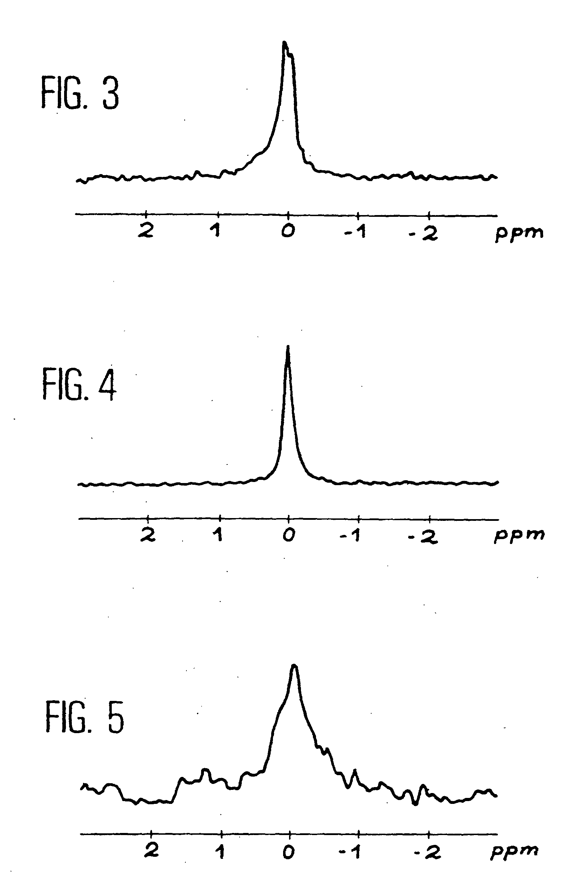

La figure 3 représente le spectre correspondant au tube a (DMPC seule).

La figure 4 représente le spectre correspondant au tube d (mélange DMPC/cyclodextrine ; 15.10⁻³/2,5.10⁻³ mol/L).

La figure 5 représente le spectre correspondant au tube b (mélange DMPC/cyclodextrine de l'art antérieur).

L'existence de petites vésicules unilamellaires de DMPC dans le tube a est confirmée sur le spectre de la figure 3, par la présence des deux pics très fins vers 0 ppm correspondant aux phosphores situés à l'intérieur et à l'extérieur des vésicules.

Le spectre de la figure 4 qui correspond à l'échantillon d, parfaitement transparent, se réduit à un seul pic fin centré à 0 ppm, indiquant la présence d'agrégats plus petits que les vésicules unilamellaires du tube a. Le spectre correspondant au tube "témoin" b indique la formation de vésicules plus grandes que les vésicules de départ. Il n'y a pas de réorganisation du milieu avec cette cyclodextrine.

Dans le tube c, la quantité de cyclodextrine de l'invention est trop faible par rapport à la quantité de DMPC pour conduire à une solution transparente comme dans le tube d. On obtient un mélange biphasique.

Le diamètre hydrodynamique moyen des agrégats mixtes de l'échantillon d a été mesuré par diffusion quasi-élastique de la lumière. La valeur du diamètre moyen (DM) calculée selon l'approximation de Stokes-Einstein basée sur le modèle des sphères parfaites sans interactions est de 13 nm (130 Å).

On examine ensuite l'échantillon d par diffusion de neutrons.

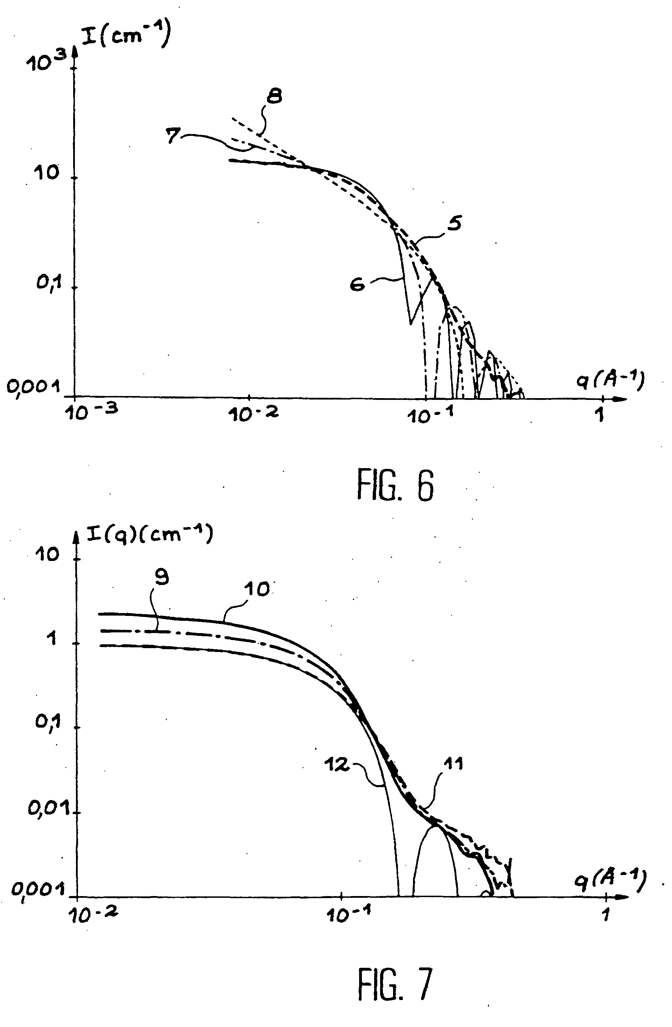

La figure 6 illustre le spectre de diffusion obtenu (spectre 5). Sur la figure 6, on a également représenté les spectres théoriques simulant des sphères (spectre 6), des cylindres (spectre 7) ou des lamelles (spectre 8) formés à partir du mélange DMPC/cyclodextrine de l'exemple 1 avec le rapport 15.10⁻³/2,5.10⁻³ mol/L dans D₂O. La superposition du spectre 6 simulant des sphères avec le spectre expérimental 5 prouve la forme sphérique des agrégats mixtes DMPC/cyclodextrine de l'exemple 1. Ces systèmes sphériques mixtes présentent à la surface des cavités de cyclodextrines susceptibles d'inclure des molécules actives hydrophobes. Le spectre théorique simulant les sphères donne un diamètre moyen de 10,8 nm (108 Å).

### Exemple 4: Préparation de complexes d'inclusion du composé mono-6-(cholest-5-èn-3α-ylamido)succinylamido-6-désoxy-2,2',2'',2''',2'''', 2''''',2'''''',6',6'',6''',6'''',6''''',6''''''-tridéca-O-méthyl-cyclomaltoheptaose avec diverses molécules actives hydrophobes.

A une solution aqueuse de nanoparticules de mono-6-(cholest-5-èn-3α-ylamido)succinylamido-6-désoxy-2,2',2'',2''',2'''',2''''',2'''''', 6',6'',6''',6'''', 6''''',6''''''-tridéca-*O*-méthyl-cyclomaltoheptaose obtenue comme dans l'exemple 2, on ajoute simplement le composé hydrophobe soit directement, soit en solution dans un solvant organique approprié, par exemple l'acétone, qu'on laisse évaporer lentement à l'air libre.

Différentes molécules actives hydrophobes ont été testées et se sont révélées capables de former des complexes d'inclusion avec la cyclodextrine de l'exemple 1. Ainsi, on a solubilisé en milieu aqueux par formation d'un complexe d'inclusion l'acide 16-iodo-3-méthylhexadécanoïque, un acide gras utilisé comme agent de contraste pour l'imagerie médicale qui avait déjà pu être solubilisé dans des cyclodextrines, comme il est décrit dans FR-A-2 726 765 [12].

La figure 7 représente les spectres de diffusion de neutrons obtenus avec :
- la solution de nanoparticules de cyclodextrine de l'exemple 1 et l'acide gras ( 1 : 0,5 éq. mol) (spectre 9) ;
- la solution de nanoparticules de cyclodextrine de l'exemple 1 et l'acide gras (1 : 1 éq. mol) (spectre 10) ; et
- la solution de nanoparticules de cyclodextrine seule (spectre 11).

Sur cette figure, on a également représenté le spectre théorique (Spectre 12) simulant les sphères.

Sur cette figure, on voit que l'incorporation des molécules d'acide gras dans les nanoparticules de la cyclodextrine de l'exemple 1 entraîne des modifications nettes au niveau des spectres de diffusion. L'intensité I(q) se trouve augmentée. L'intensité I(q) est proportionnelle au volume des nanoparticules. La présence de molécules supplémentaires (molécules d'acide gras) dans les nanoparticules a pour effet d'augmenter la valeur du contraste et donc de l'intensité.

L'incorporation dans les nanoparticules de mono-6-(cholest-5-èn-3α-ylamido)succinylamido-6-desoxy-2,2',2'',2''',2'''',2''''',2'''''',6',6'',6''',6'''',6' '''',6''''''-tridéca-*O*-méthyl-cyclomaltoheptaose des composés hydrophobes suivants :
- la dothiépine (neurotrope),
- le chloramphénicol (bactériostatique),
- la vitamine A, et
- l'esculine (tonique de la paroi vasculaire),
a également été mise en évidence par diffusion de neutrons.

### Références citées.

[1] : D. Duchêne "Pharmaceutical Applications of Cyclodextrins » paru dans « Cyclodextrins and their industrial uses", Editions de Santé, Paris 1987, pp. 213-257.
[2] : A. Yabe et coll, Thin Solid Films, (1988), 160, pp. 33-41.
[3] : L. Jullien et coll, « J. Chem. Soc. Perkin Trans 2, 1993, pp. 1011-1022.
[4] : A. Gulik et coll dans Langmuir (1998), 14, pp. 1050-1057.
[5] : FR-A-2 736 056.
[6] : J. Chem. Soc. Perkin Trans 2,(1998), pp. 2638-2646.
[7] : Uekama et Irie dans Chemical Review (1998), 98, pp. 2045-2076.
[8] : Tetrahedron Lett. (1993), 34, pp 2457-2460.
[9] : J. Chem. Soc. Perkin Trans 2 (1995), pp 723-730.
[10] : Tetrahedron Lett. (1977), pp. 1977-1980.
[11] : "Liposomes: a practical approach", R.R.C. New Ed., IRL Press, Oxford University Press, 1990).
[12] : FR-A-2 726 765.

## Revendications

1. Dérivé amphiphile de cyclodextrine répondant à la formule : 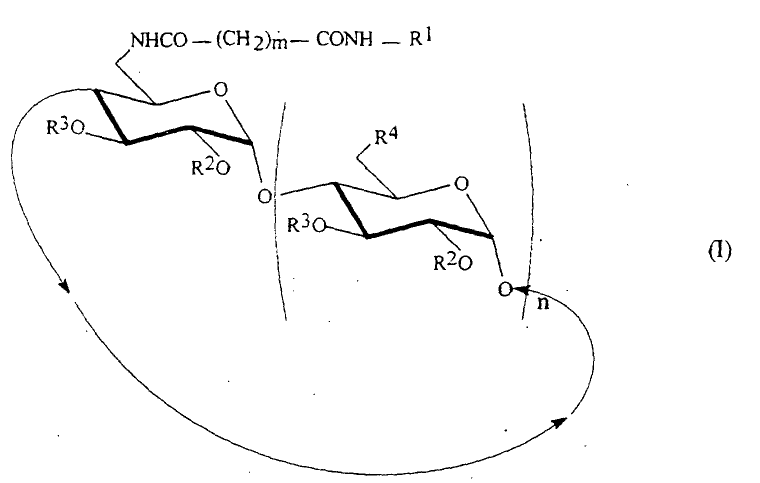 dans laquelle :
- R¹ représente un groupe dérivé d'un stéroïde,
- R² représente un groupe alkyle ou aryle éventuellement substitué,
- R³ représente H ou R²,
- tous les R⁴ représentent OR², ou
- l'un des R⁴ représente -NHCO(CH₂)ₘCONHR¹ et les autres R⁴ représentent OR² à condition qu'il y ait au moins une unité glucose avec R⁴ représentant OR² entre les deux unités glucose comportant le substituant ―NHCO―(CH₂)ₘ―CONH―R¹,
- m est un nombre entier allant de 1 à 8, et
- n est égal à 5, 6 ou 7.

2. Dérivé de cyclodextrine selon la revendication 1 dans lequel R¹ représente le groupe de formule :

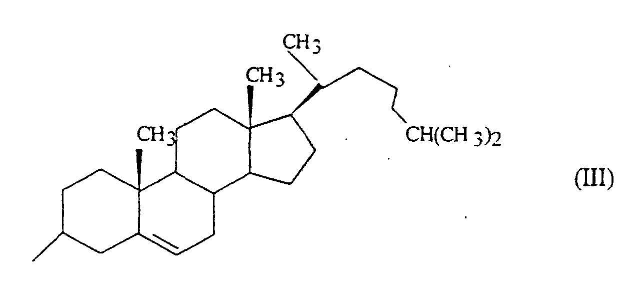

3. Dérivé de cyclodextrine selon la revendication 1 ou 2, dans lequel tous les R⁴ représentent OR².

4. Dérivé de cyclodextrine selon l'une quelconque des revendications 1 à 3, dans lequel R² représente le groupe méthyle et R³ représente un atome d'hydrogène.

5. Dérivé de cyclodextrine selon l'une quelconque des revendications 1 à 4, dans lequel n est égal à 6.

6. Dérivé de cyclodextrine selon l'une quelconque des revendications 1 à 5, dans lequel m est égal 2.

7. Mono-6-(cholest-5-èn-3α-ylamido)succinylamido-6-désoxy-2,2',2'',2''',2'''', 2''''', 2'''''',6',6'',6''',6'''',6''''',6''''''-tridéca-O-méthyl-cyclomaltoheptaose.

8. Procédé de préparation d'un dérivé de cyclodextrine de formule : 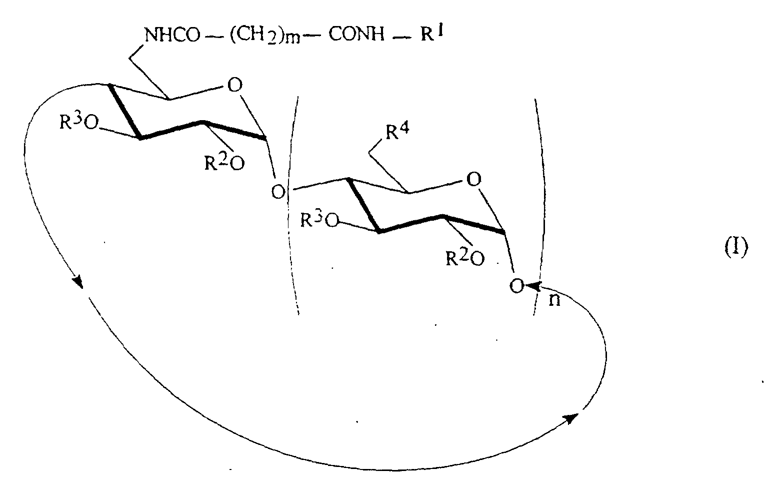 dans laquelle :
- R¹ représente un groupe dérivé d'un stéroïde,
- R² représente un groupe alkyle ou aryle éventuellement substitué,
- R³ représente H,
- tous les R⁴ représentent OR², ou
- l'un des R⁴ représente -NHCO(CH₂)ₘCONHR¹ et les autres R⁴ représent OR² à condition qu'il y ait au moins une unité glucose avec R⁴ représentant OR² entre les deux unités glucose comportant le substituant ―NHCO―(CH₂)ₘ―CONH―R¹,
- m est un nombre entier allant de 1 à 8, et
- n est égal à 5, 6 ou 7,
qui comprend les étapes suivantes :
a) faire réagir un dérivé de formule : 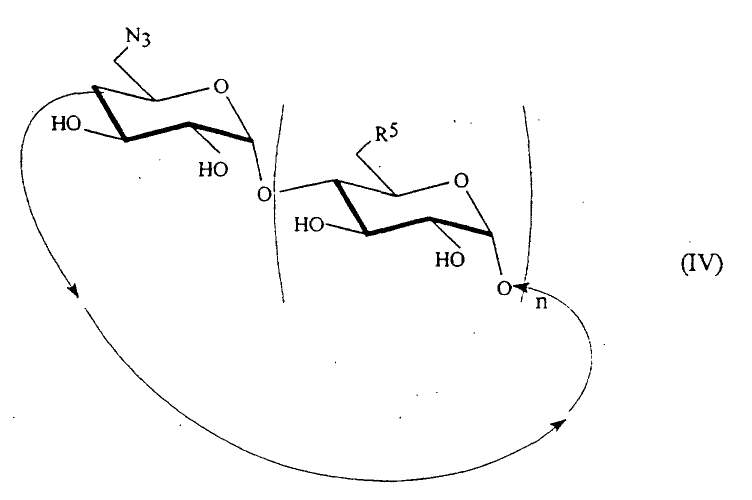 dans laquelle tous les R⁵ représentent OH, ou l'un des R⁵ represente ―N₃ et les autres R⁵ représentent OH à condition qu'il y ait au moins une unité glucose avec R⁵ représentant OH entre les deux unités glucose comportant le substituant N₃, et n est égal à 5, 6 ou 7,
avec un sulfate de dialkyle SO₄R²₂ avec R² ayant la signification donnée ci-dessus, en milieu basique pour obtenir le dérivé de cyclodextrine de formule : 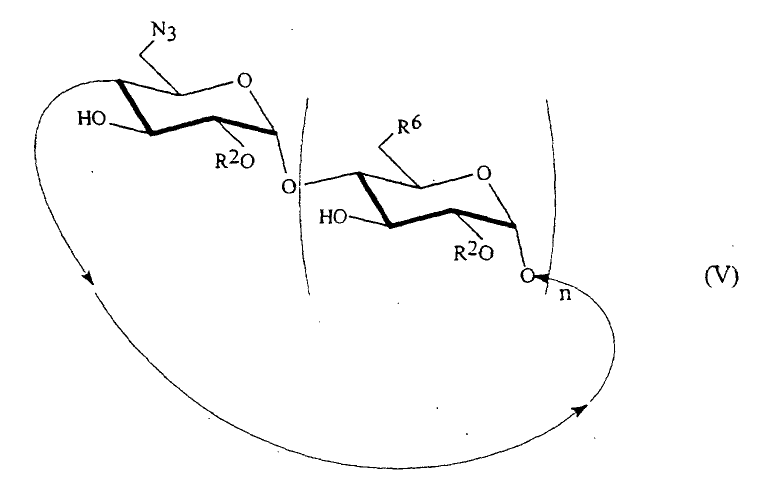 dans laquele tous les R⁶ représentent OR², ou l'un des R⁶ représente N₃ et les autres R⁶ représentent OR², et R² et n sont tels que définis ci-dessus,
b) effectuer une réaction de Staudinger sur le dérivé de formule (V) à l'aide de triphénylphosphine et d'ammoniaque pour convertir N₃ en NH₂ et obtenir le dérivé de formule : 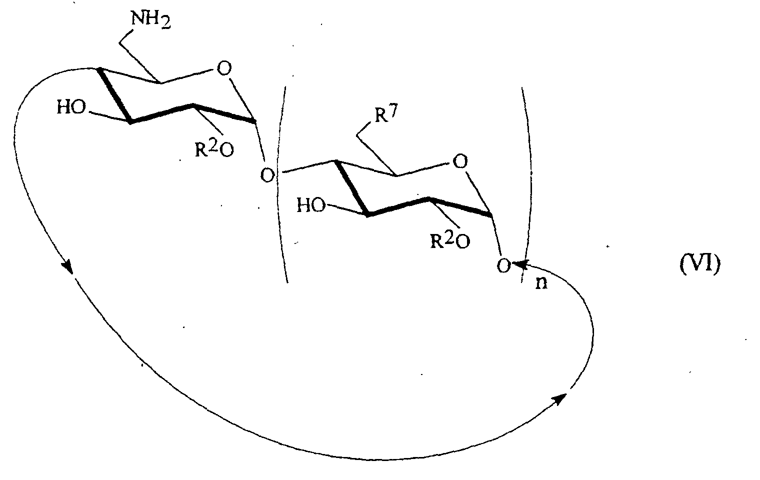 dans laquelle tous les R⁷ représentent OR², ou l'un des R⁷ représente NH₂ et les autres R⁷ représentent OR², et R² et n sont tels que définis ci-dessus,
c) faire réagir le dérivé de formule (VI) avec un anhydride d'acide de formule : 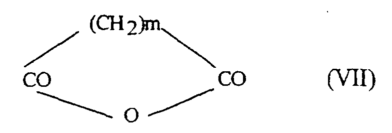 où m est tel que défini ci-dessus, pour obtenir le dérivé de formule : 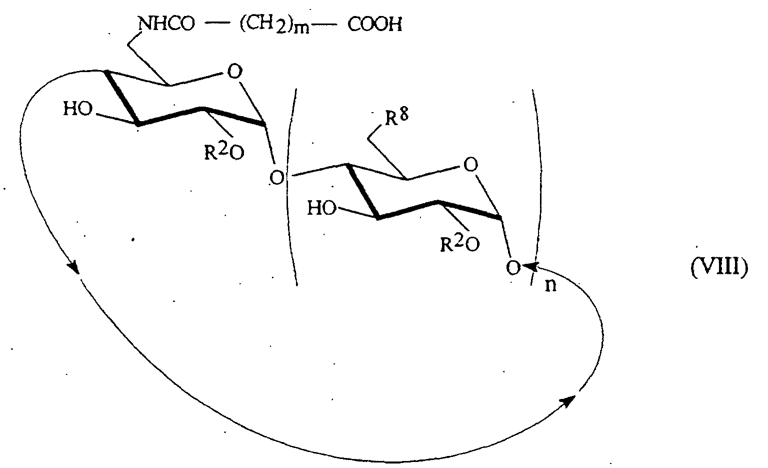 dans laquelle tous les R⁸ représentent OR² ou l'un des R⁸ représente ―NHCO―(CH₂)ₘ―COOH et les autres R⁸ représentent OR², et R², m et n sont tels que définis ci-dessus, et
d) faire réagir le dérivé de formule (VIII) avec un composé de formule NH₂-R¹ pour obtenir le dérivé de cyclodextrine de formule (I) défini ci-dessus.

9. Procédé de préparation d'un dérivé de cyclodextrine de formule : 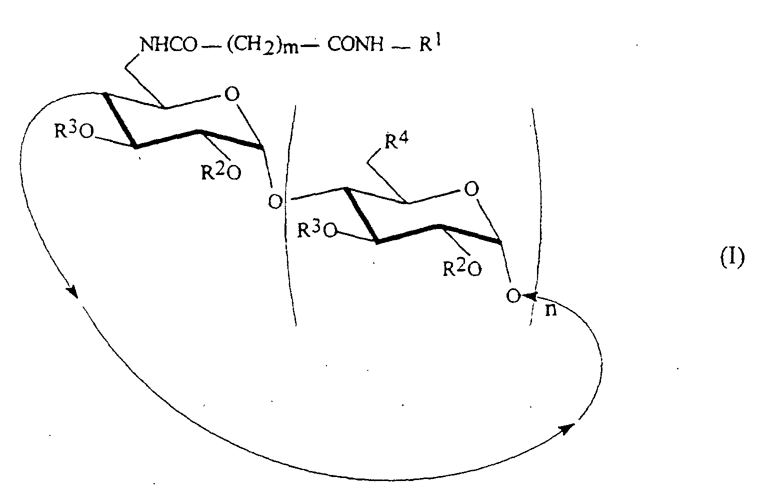 dans laquelle :
- R¹ représente un groupe dérivé d'un stéroïde,
- R² représente un groupe alkyle ou aryle éventuellement substitué,
- R³ représente R²,
- tous les R⁴ représentent OR², ou
- l'un des R⁴ représente -NHCO(CH₂)ₘCONHR¹ et les autres R⁴ représentent OR² à condition qu'il y ait au moins une unité glucose avec R⁴ représentant OR² entre les deux unités glucose comportant le substituant ―NHCO―(CH₂)ₘ―CONH―R¹,
- m est un nombre entier allant de 1 à 8, et
- n est égal à 5., 6 ou 7,
qui comprend les étapes suivantes :
a) faire réagir un dérivé de formule : 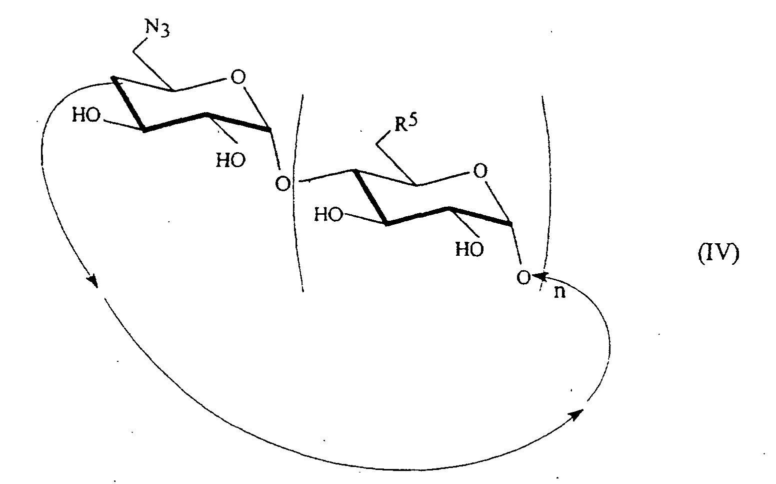 dans laquelle tous les R⁵ représentent OH, ou l'un des R⁵ represente ―N₃ et les autres R⁵ représentent OH à condition qu'il y ait au moins une unité glucose, avec R⁵ représentant OH entre les deux unités glucose comportant le substituant N₃, et n est égal à 5, 6 ou 7,
avec un iodo alcane de formule IR² dans laquelle R² a la signification donnée ci-dessus, en présence de NaH pour pour obtenir le dérivé de cyclodextrine de formule : 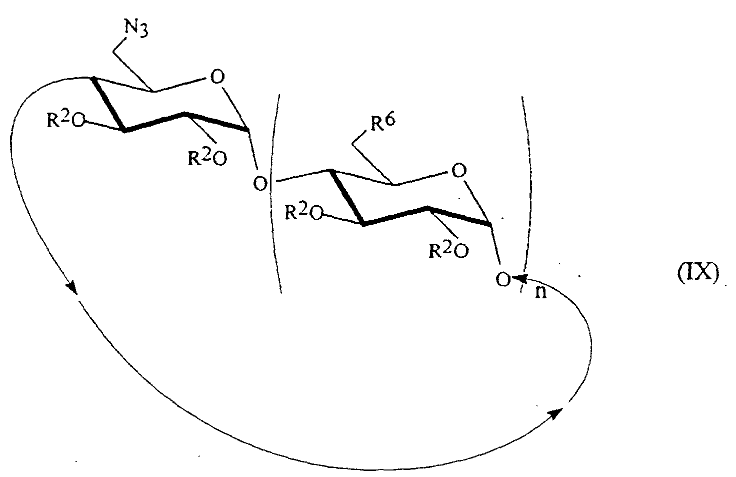 dans laquelle tous les R⁶ représentent OR², ou l'un des R⁶ représente N₃ et les autres R⁶ représentent OR², et R² et n sont tels que définis ci-dessus,
b) effectuer une réaction de Staudinger sur le dérivé de formule (IX) à l'aide de triphénylphosphine et d'ammoniaque pour convertir N₃ en NH₂ et obtenir le dérivé de formule : 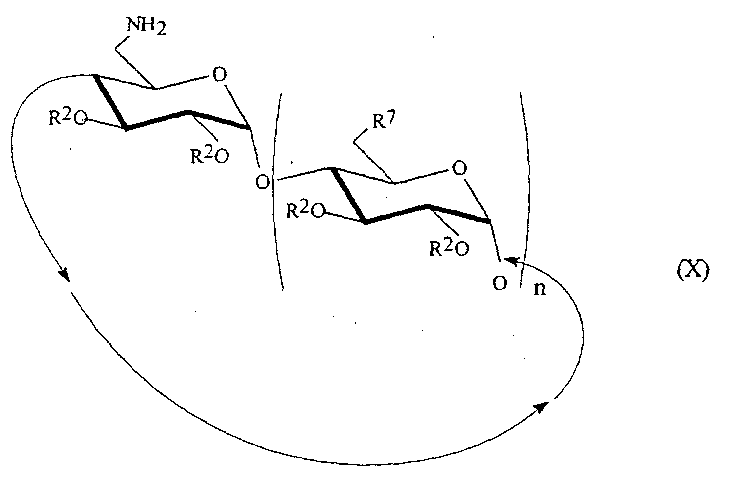 dans laquelle tous les R⁷ représentent OR², ou l'un des R⁷ représente NH₂ et les autres R⁷ représentent OR², et R² et n sont tels que définis ci-dessus,
c) faire réagir le dérivé de formule (X) avec un anhydride d'acide de formule : 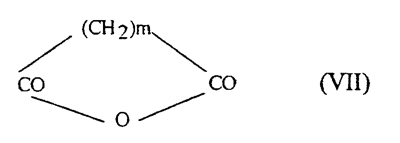 où m est tel que défini ci-dessus, pour obtenir le dérivé de formule : 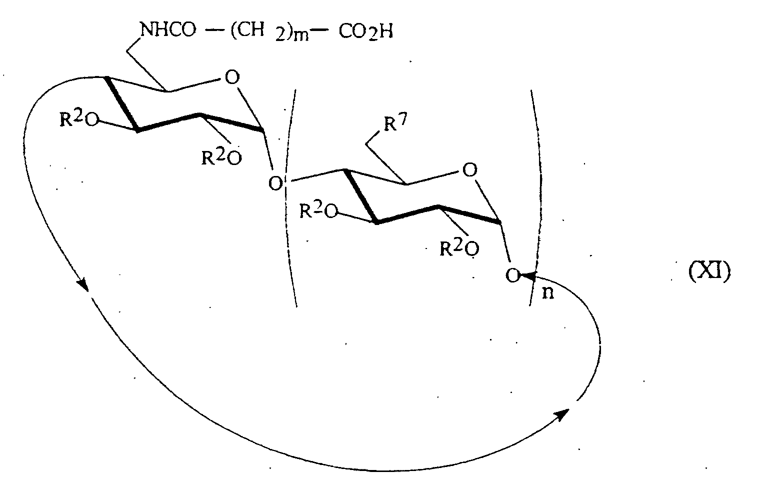 dans laquelle tous les R⁷ représentent OR² ou l'un des R⁷ représente ―NHCO―(CH₂)ₘ―COOH et les autres R⁷ représentent OR², et R², m et n sont tels que définis ci-dessus, et
d) faire réagir le dérivé de formule (XI) avec un composé de formule NH₂-R¹ pour obtenir le dérivé de cyclodextrine de formule (I) défini ci-dessus.

10. Complexe d'inclusion d'un dérivé de cyclodextrine selon l'une quelconque des revendications 1 à 7, et d'un composé hydrophobe.

11. Complexe selon la revendication 10, dans lequel le composé hydrophobe est choisi parmi les stéroïdes, les neurotropes., les bactériostatiques, les vitamines, les toniques de la paroi vasculaire et les agents de contraste.

12. Complexe selon la revendication 10, dans lequel le composé hydrophobe est choisi parmi l'acide 16-iodo-3-méthylhexadécanoïque, la dothiépine, le chloramphénicol, la vitame A et l'esculine.

13. Solution aqueuse de nanoparticules d'un dérivé de cyclodextrine selon l'une quelconque des revendications 1 à 7 ou d'un complexe d'inclusion selon l'une quelconque des revendications 10 à 12.

14. Système de tensioactifs organisé comprenant un dérivé de cyclodextrine selon l'une quelconque des revendications 1 à 7 ou un complexe d'inclusion selon l'une quelconque des revendications 10 à 12.

15. Système selon la revendication 14 dans lequel le tensioactif est un phospholipide.

16. Solution aqueuse comprenant en solution un système mixte formé à partir de vésicules de phospholipides ou de protéines membranaires, et d'au moins un dérivé de cyclodextrine selon l'une quelconque des revendications 1 à 7 ou d'au moins un complexe d'inclusion selon l'une quelconque des revendications 10 à 12.

## Patentansprüche

1. Amphiphiles Cyclodextrin-Derivat, das der Formel entspricht: 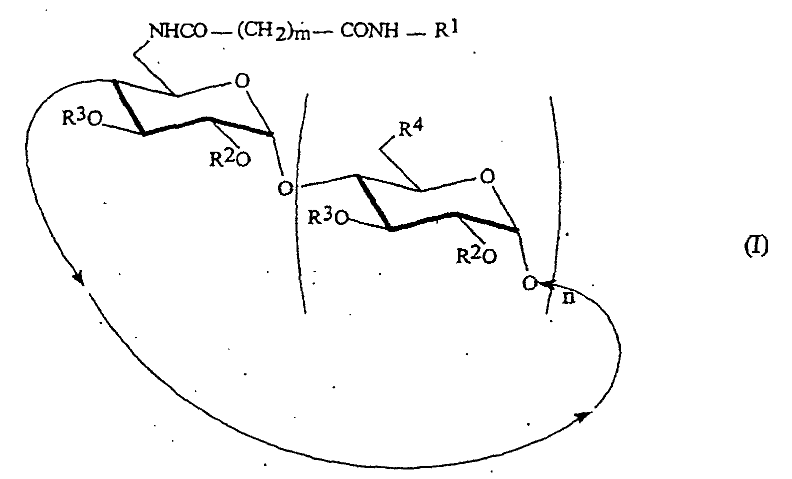 worin bedeuten:
- R¹ eine von einem Steroid abgeleitete Gruppe,
- R² eine gegebenenfalls substituierte Alkyl- oder Arylgruppe,
- R³ H oder R²,
- alle R⁴-Reste für OR² stehen oder
- einer der R⁴-Reste steht für -NHCO(CH₂)ₘCONHR¹ und die anderen R⁴-Reste für OR² stehen, mit der Maßgabe, dass mindestens eine Glucose-Einheit, worin R⁴ für OR² steht, zwischen den beiden Glucose-Einheiten vorliegt, die den Substituenten -NHCO-(CH₂)ₘCONH-R¹ aufweist;
- m steht für eine ganze Zahl von 1 bis 8 und
- n steht für die Zahl 5, 6 oder 7.

2. Cyclodextrin-Derivat nach Anspruch 1, worin R¹ eine Gruppe der Formel darstellt:

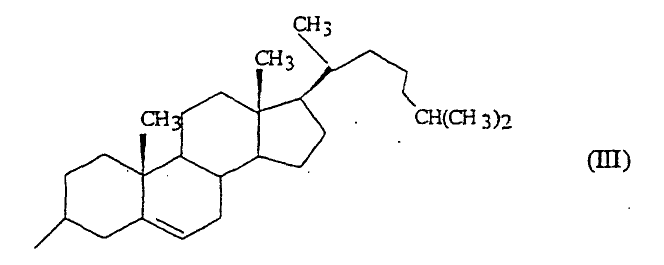

3. Cyclodextrin-Derivat nach Anspruch 1 oder 2, worin alle R⁴-Reste OR² darstellen.

4. Cyclodextrin-Derivat nach einem der Ansprüche 1 bis 3, worin R² die Methylgruppe und R³ ein Wasserstoffatom darstellen.

5. Cyclodextrin-Derivat nach einem der Ansprüche 1 bis 4, worin n für die Zahl 6 steht.

6. Cyclodextrin-Derivat nach einem der Ansprüche 1 bis 5, worin n für die Zahl 2 steht.

7. Mono-6-(cholest-5-en-3α-ylamido)succinylamido-6-desoxy-2,2',2'',2''',2'''',2''''',2'''''',6',6'',6''',6'''',6''''',6''''''-trideca-O-methyl-cyclomaltoheptaose.

8. Verfahren zur Herstellung eines Cyclodextrin-Derivats der Formel 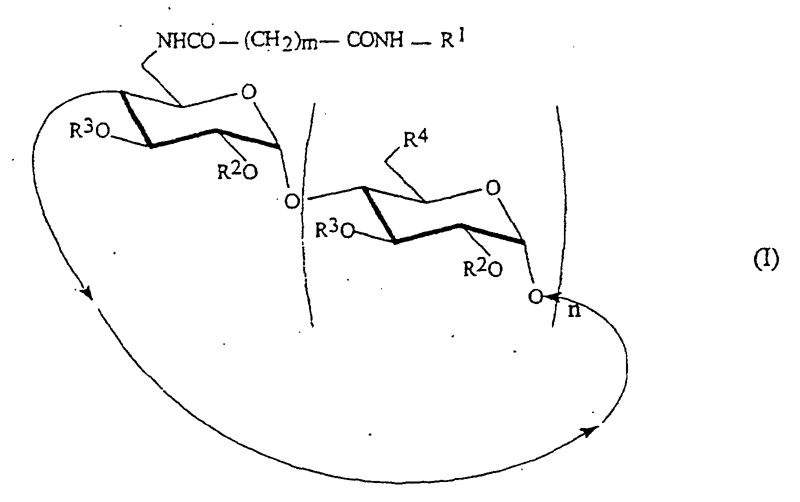 worin bedeuten:
- R¹ eine von einem Steroid abgeleitete Gruppe,
- R² eine gegebenenfalls substituierte Alkyl- oder Arylgruppe,
- R³ H,
- alle R⁴-Reste für OR² stehen oder
- einer der R⁴-Reste steht für -NHCO(CH₂)ₘCONHR¹ und die anderen R⁴-Reste für OR² stehen, mit der Maßgabe, dass mindestens eine Glucose-Einheit, worin R⁴ für OR² steht, zwischen den beiden Glucose-Einheiten vorliegt, die den Substituenten -NHCO-(CH₂)ₘCONH-R¹ aufweisen;
- m steht für eine ganze Zahl von 1 bis 8 und
- n steht für die Zahl 5, 6 oder 7, das die folgenden Stufen umfasst:
a) Reagierenlassen eines Derivats der Formel: 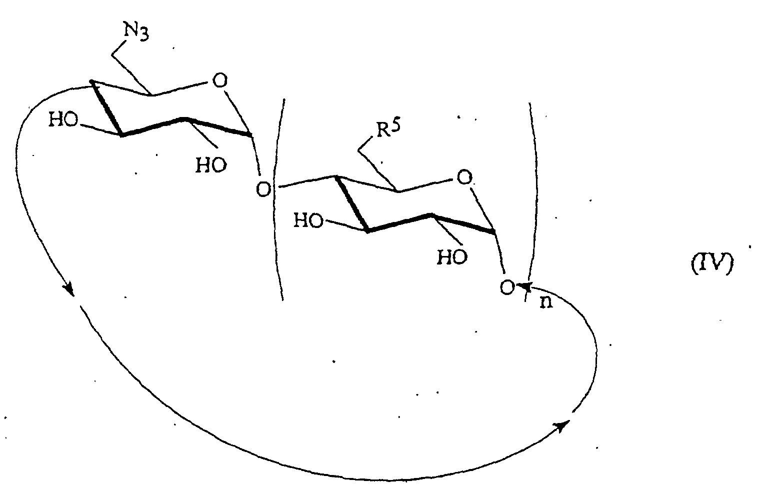 in der alle R⁵-Reste OH darstellen oder einer der R⁵-Reste -N₃ darstellt und die anderen R⁵-Reste OH darstellen, mit der Maßgabe, dass mindestens eine Glucose-Einheit, in der R⁵ OH darstellt, zwischen den beiden Glucose-Einheiten vorliegt, die den Substituenten N₃ aufweisen, und n für die Zahl 5, 6 oder 7 steht,
mit einem Dialkylsulfat SO₄R²₂, worin R² die oben angegebene Bedeutung hat, in einem basischen Medium zur Herstellung des Cyclodextrin-Derivats der Formel: 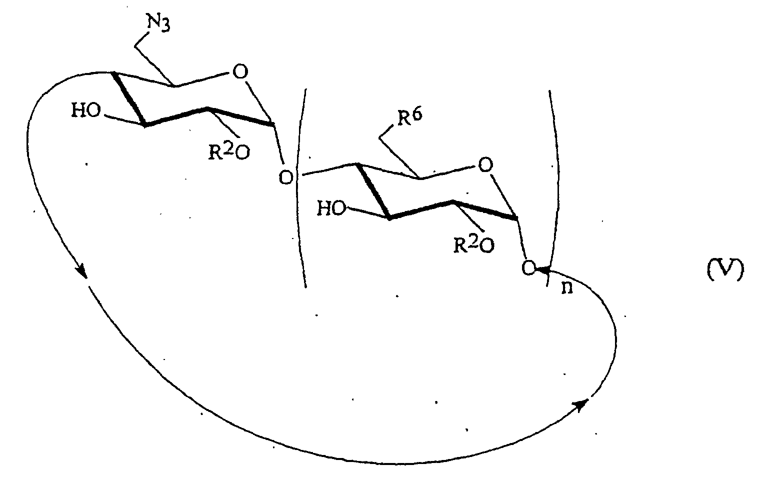 in der alle R⁶-Reste OR² darstellen oder einer der R⁶-Reste N₃ darstellt und die anderen R⁶-Reste OR² darstellen und in der R² und n wie oben definiert sind,
b) Durchführung einer Staudinger-Reaktion mit dem Derivat der Formel (V) durch Verwendung von Triphenylphosphin und Ammoniak, um N₃ in NH₂ umzuwandeln und zur Herstellung des Derivats der Formel 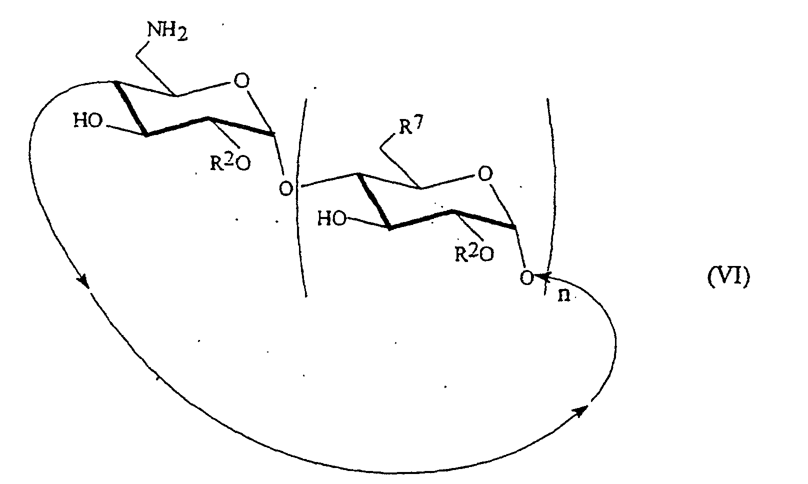 in der alle R⁷-Reste OR² darstellen oder einer der R⁷-Reste NH₂ darstellt und die anderen R⁷-Reste OR² darstellen und in der R² und n wie oben definiert sind,
c) Reagierenlassen des Derivats der Formel (VI) mit einem Säureanhydrid der Formel: 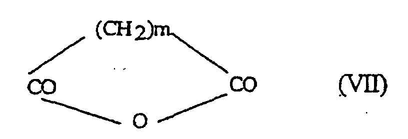 in der m wie oben definiert ist, zur Herstellung des Derivats der Formel: 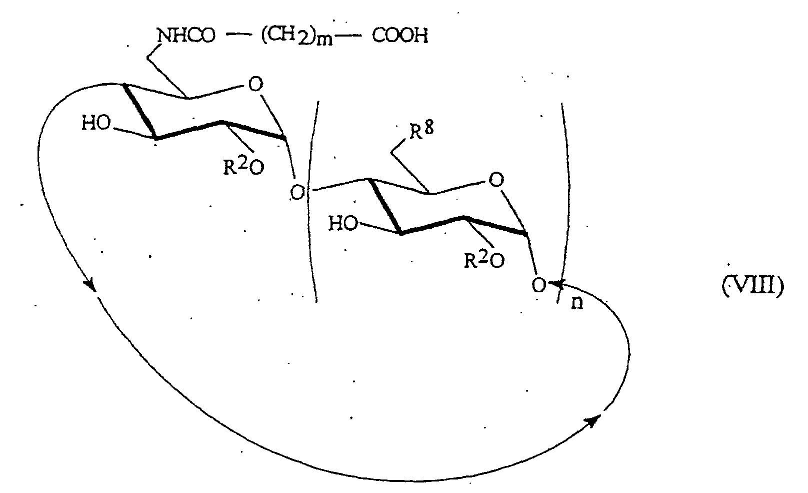 in der alle R⁸-Reste OR² darstellen oder einer der R⁸-Reste -NHCO-(CH₂)ₘCOOH darstellt und die anderen R⁸-Reste OR² darstellen und worin R², m und n wie oben definiert sind, und
d) Reagierenlassen des Derivats der Formel (VIII) mit einer Verbindung der Formel NH₂-R¹ zur Herstellung des Cyclodextrin-Derivats der Formel (I), wie oben definiert.

9. Verfahren zur Herstellung eines Cyclodextrin-Derivats der Formel: 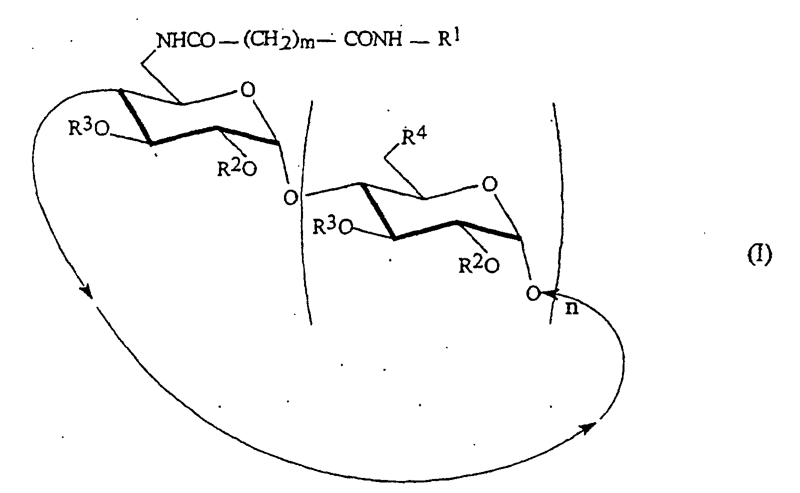 worin
- R¹ steht für eine von einem Steroid abgeleitete Gruppe,
- R² steht für eine gegebenenfalls substituierte Alkyl- oder Arylgruppe,
- R³ steht für R²,
- alle R⁴-Reste stehen für OR² oder
- einer der R⁴-Reste steht für -NHCO(CH₂)ₘCONHR¹ und die anderen
R⁴-Reste stehen für OR² mit der Maßgabe, dass mindestens eine Glucose-Einheit, worin R⁴ für OR² steht, zwischen den beiden Glucose- Einheiten vorliegt, die den Substituenten -NHCO-(CH₂)ₘCONH-R¹ aufweisen;
- m steht für eine ganze Zahl von 1 bis 8 und
- n steht für die Zahl 5, 6 oder 7
das die folgenden Stufen umfasst:
a) Reagierenlassen eines Derivats der Formel: 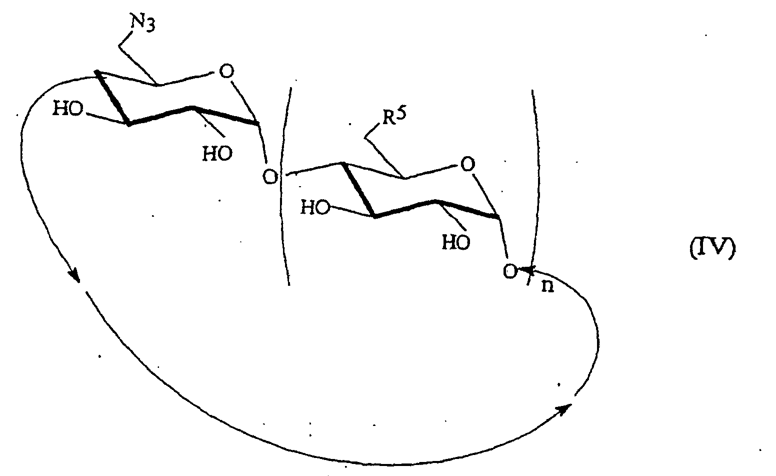 in der alle R⁵-Reste für OH stehen oder einer der R⁵-Reste für -N₃ steht und die anderen R⁵-Reste für OH stehen, mit der Maßgabe, dass mindestens eine Glucose-Einheit, in der R⁵ für OH steht, zwischen den beiden Glucose-Einheiten vorliegt, die den Substituenten N₃ aufweisen, und n für die Zahl 5, 6 oder 7 steht,
mit einem lodalkan der Formel IR², worin R² die oben angegebene Bedeutung hat, in Gegenwart von NaH zur Herstellung des Cyclodextrin-Derivats der Formel: 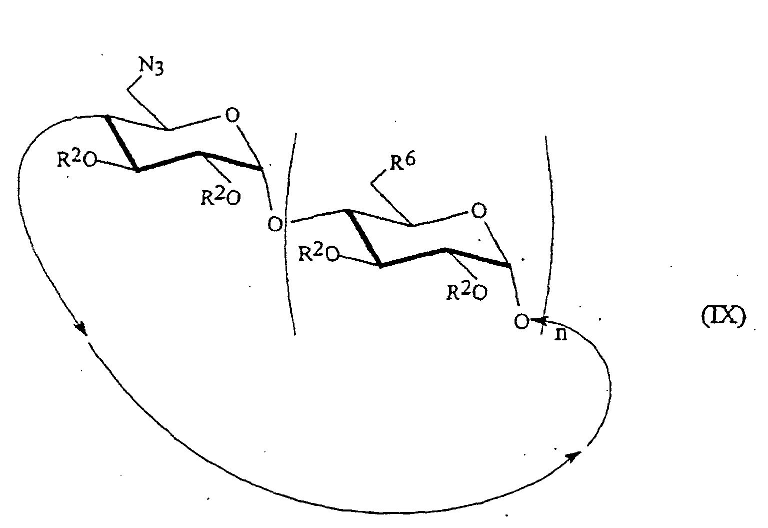 in der alle R⁶-Reste für OR² stehen oder einer der R⁶-Reste für N₃ und die anderen R⁶-Reste für OR² stehen und worin R² und n wie oben definiert sind,
b) Durchführung einer Staudinger-Reaktion mit dem Derivat der Formel (IX) durch Verwendung von Triphenylphosphin und Ammoniak, um N₃ in NH₂ umzuwandeln und das Derivat der Formel herzustellen: 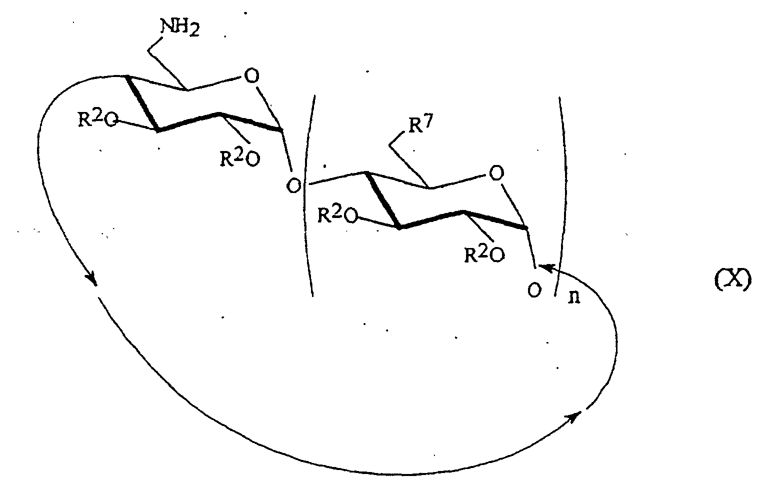 worin alle R⁷-Reste für OR² stehen oder einer der R⁷-Reste für NH₂ und die anderen R⁷-Reste für OR² stehen und worin R² und n wie oben definiert sind,
c) Reagierenlassen des Derivats der Formel (X) mit einem Säureanhydrid der Formel: 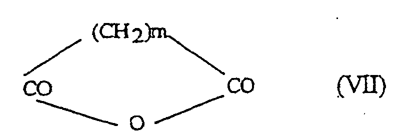 worin m wie oben definiert ist, zur Herstellung des Derivats der Formel: 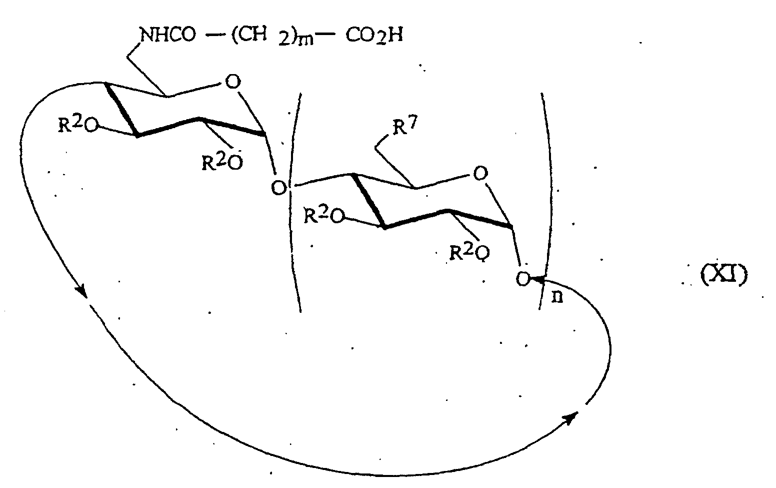 worin alle R⁷-Reste für OR² stehen oder einer der R⁷-Reste für -NHCO-(CH₂)ₘCOOH steht und die anderen R⁷-Reste für OR² stehen und worin R², m und n wie oben definiert sind, und
d) Reagierenlassen des Derivats der Formel (XI) mit einer Verbindung der Formel NH₂-R¹ zur Herstellung des Cyclodextrin-Derivats der Formel (I) wie oben definiert.

10. Einschlusskomplex aus einem Cyclodextrin-Derivat nach einem der Ansprüche 1 bis 7 und einer hydrophoben Verbindung.

11. Komplex nach Anspruch 10, in der die hydrophobe Verbindung ausgewählt ist aus der Gruppe der Steroide, der Neurotrope, der bakteriostatischen Agentien, der Vitamine, der Tonika für die Gefäßwand und der Kontrastmittel.

12. Komplex nach Anspruch 10, in der die hydrophobe Verbindung ausgewählt ist aus der Gruppe 16-Iodo-3-methylhexadecansäure, Dothiepin, Chloramphenicol, Vitamin A und Esculin.

13. Wässrige Lösung von Nanoteilchen eines Cyclodextrin-Derivats nach einem der Ansprüche 1 bis 7 oder eines Einschluss-Komplexes nach einem der Ansprüche 10 bis 12.

14. Tensid-System, das so aufgebaut ist, dass es umfasst ein Cyclodextrin-Derivat nach einem der Ansprüche 1 bis 7 oder einen Einschluss-Komplex nach einem der Ansprüche 10 bis 12.

15. System nach Anspruch 14, in dem das Tensid ein Phospholipid ist.

16. Wässrige Lösung, die ein gemischtes System in Lösung umfasst, das hergestellt worden ist aus Phospholipid- oder Proteinmembran-Vesikula und mindestens einem Cyclodextrin-Derivat nach einem der Ansprüche 1 bis 7 oder mindestens einem Einschluss-Komplex nach einem der Ansprüche 10 bis 12.

## Claims

1. Amphiphilic cyclodextrin derivative complying with the formula: 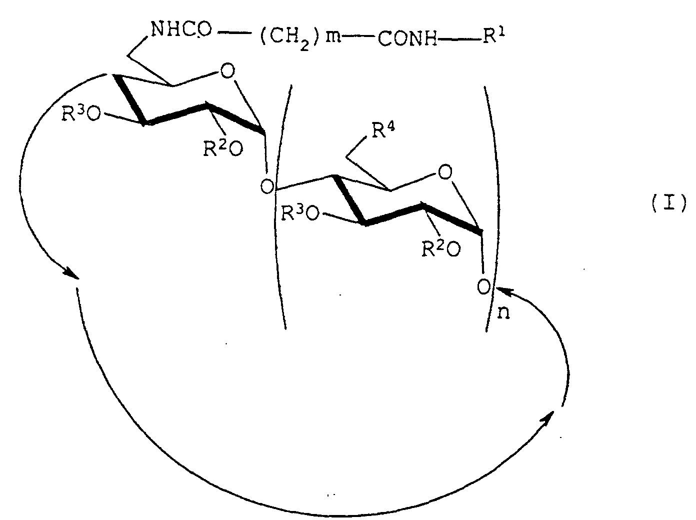 wherein:
- R¹ represents a group derived from a steroid,
- R² represents an alkyl or aryl group, substituted if applicable,
- R³ represents H or R²,
- all the R⁴ represent OR², or
- one of the R⁴ represents -NHCO(CH₂)ₘCONHR¹, and the other R⁴ represent OR² provided that there is at least one glucose unit where R⁴ represents OR² between the two glucose units comprising the substituent ―NHCO―(CH₂)ₘ―CONH―R¹,
- m is an integer ranging from 1 to 8, and
- n is equal to 5, 6 or 7.

2. Cyclodextrin derivative according to claim 1 wherein R¹ represents the group according to the formula:

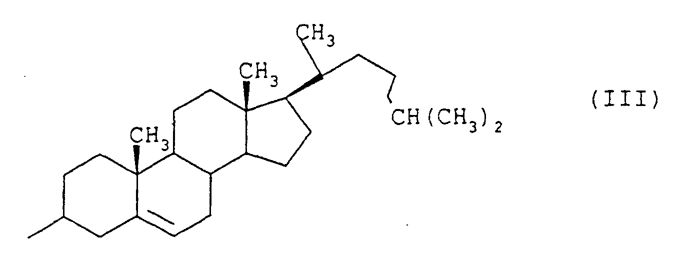

3. Cyclodextrin derivative according to claim 1 or 2, wherein all the R⁴ represent OR².

4. Cyclodextrin derivative according to any of claims 1 to 3, wherein R² represents the methyl group and R³ represents a hydrogen atom.

5. Cyclodextrin derivative according to any of claims 1 to 4, wherein n is equal to 6.

6. Cyclodextrin derivative according to any of claims 1 to 5, wherein m is equal to 2.

7. Mono-6-(cholest-5-en-3α-ylamide)succinylamide-6-deoxy-2,2',2'',2''',2'''',2''''',2'''''',6',6'',6''', 6'''',6''''',6''''''-trideca-O-methyl-cyclomaltoheptaose.

8. Method to prepare a cyclodextrin derivative according to the formula: 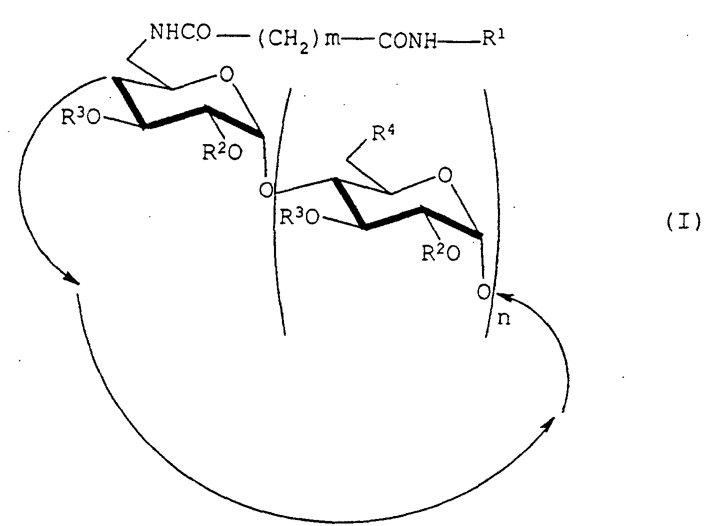 wherein:
- R¹ represents a group derived from a steroid,
- R² represents an alkyl or aryl group, substituted if applicable,
- R³ represents H,
- all the R⁴ represent OR², or
- one of the R⁴ represents -NHCO(CH₂)ₘCONHR¹, and the other R⁴ represent OR² provided that there is at least one glucose unit where R⁴ represents OR² between the two glucose units comprising the substituent ―NHCO―(CH₂)ₘ―CONH―R¹,
- m is an integer ranging from 1 to 8, and
- n is equal to 5, 6 or 7,
which comprises the following steps:
a) react a derivative according to the formula: 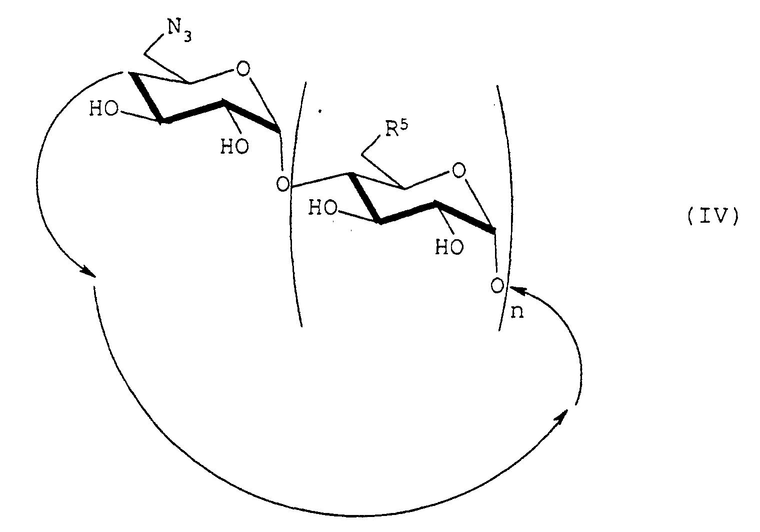 wherein all the R⁵ represent OH, or one of the R⁵ represents ―N₃ and the other R⁵ represent OH, provided that there is at least one glucose unit where R⁵ represents OH between the two glucose units comprising the N₃ substituent, and n is equal to 5, 6 or 7,
with a dialkyl sulphate SO₄R²₂ where R² has the significance given above, in a basic medium to obtain the cyclodextrin derivative according to the formula: 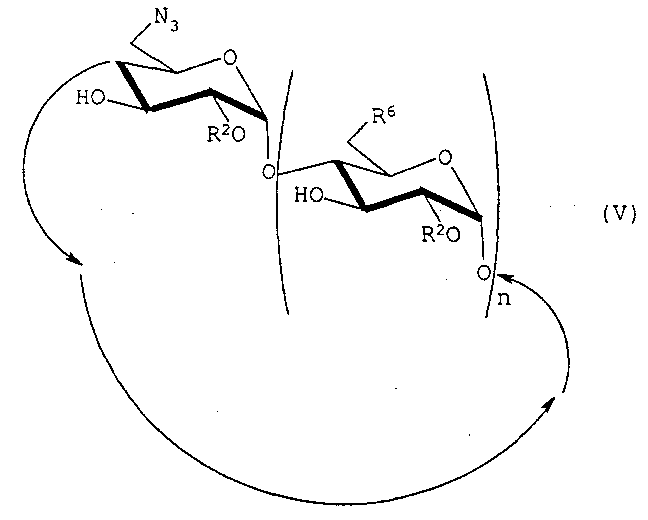 wherein all the R⁶ represent OR², or one of the R⁶ represents N₃ and the other R⁶ represent OR², and R² and n are as defined above,
b) perform a Staudinger reaction on the derivative according to formula (V) using triphenylphosphine and ammonia to convert N₃ into NH₂ and obtain the derivative according to the formula: 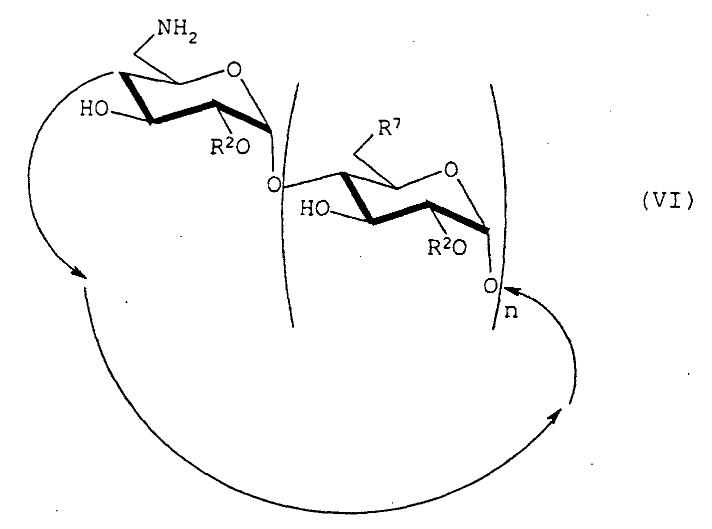 wherein all the R⁷ represent OR², or one of the R⁷ represents NH₂ and the other R⁷ represent OR², and R² and n are as defined above,
c) react the derivative according to formula (VI) with an acid anhydride according to the formula: 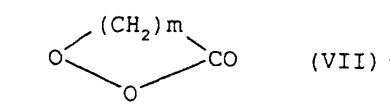 where m is as defined above, to obtain the derivative according to the formula: 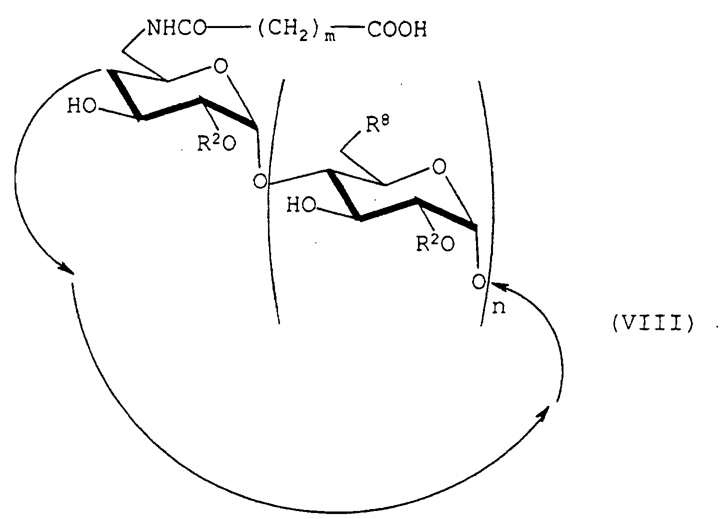 wherein all the R⁸ represent OR², or one of the R⁸ represents ―NHCO―(CH₂)ₘ―COOH and the other R⁸ represent OR², and R², m and n are as defined above, and
d) react the derivative according to formula (VIII) with a compound according to the formula NH₂-R¹ to obtain the cyclodextrin derivative according to formula (I) defined above.

9. Method to prepare a cyclodextrin derivative according to the formula: 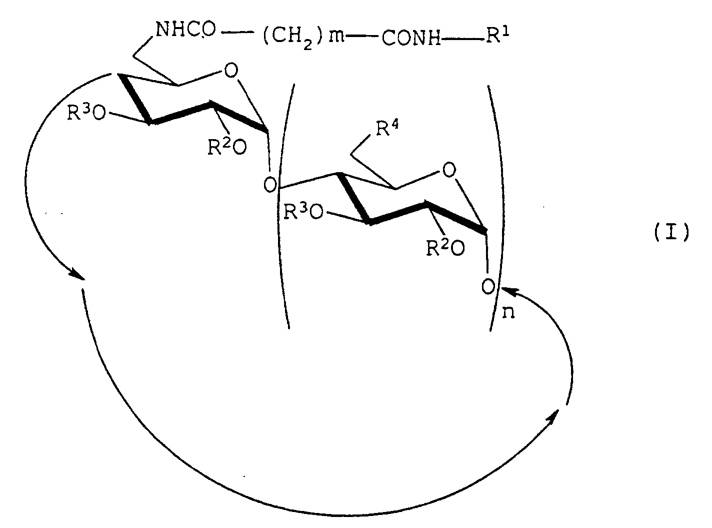 wherein:
- R¹ represents a group derived from a steroid,
- R² represents an alkyl or aryl group, substituted if applicable,
- R³ represents R²,
- all the R⁴ represent OR², or
- one of the R⁴ represents -NHCO(CH₂)ₘCONHR¹, and the other R⁴ represent OR² provided that there is at least one glucose unit where R⁴ represents OR² between the two glucose units comprising the substituent ―NHCO―(CH₂)ₘ―CONH―R¹,
- m is an integer ranging from 1 to 8, and
- n is equal to 5, 6 or 7,
which comprises the following steps:
a) react a derivative according to the formula: 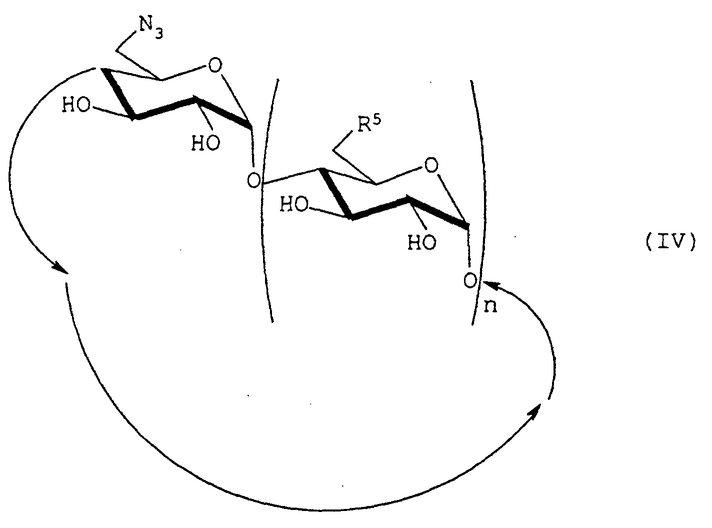 wherein all the R⁵ represent OH, or one of the R⁵ represents ―N₃ and the other R⁵ represent OH, provided that there is at least one glucose unit where R⁵ represents OH between the two glucose units comprising the N₃ substituent, and n is equal to 5, 6 or 7,
with an iodoalkane according to the formula IR² wherein R² has the significance given above, in the presence of NaH to obtain the cyclodextrin derivative according to the formula: 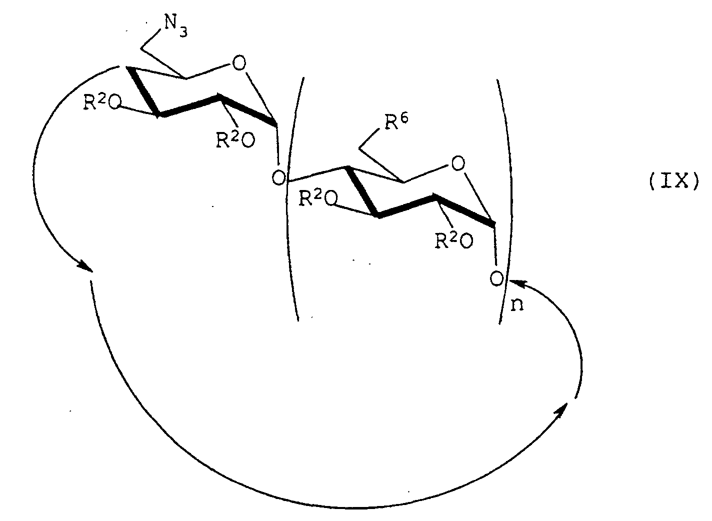 wherein all the R⁶ represent OR², or one of the R⁶ represents N₃ and the other R⁶ represent OR², and R² and n are as defined above,
b) perform a Staudinger reaction on the derivative according to formula (IX) using triphenylphosphine and ammonia to convert N₃ into NH₂ and obtain the derivative according to the formula: 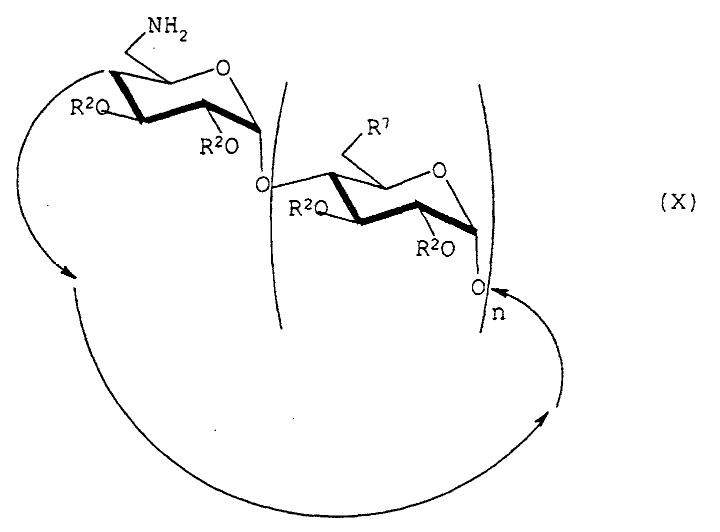 wherein all the R⁷ represent OR², or one of the R⁷ represents NH₂ and the other R⁷ represent OR², and R² and n are as defined above,
c) react the derivative according to formula (X) with an acid anhydride according to the formula: 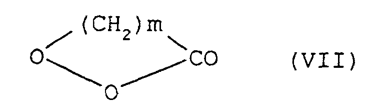 where m is as defined above, to obtain the derivative according to the formula: 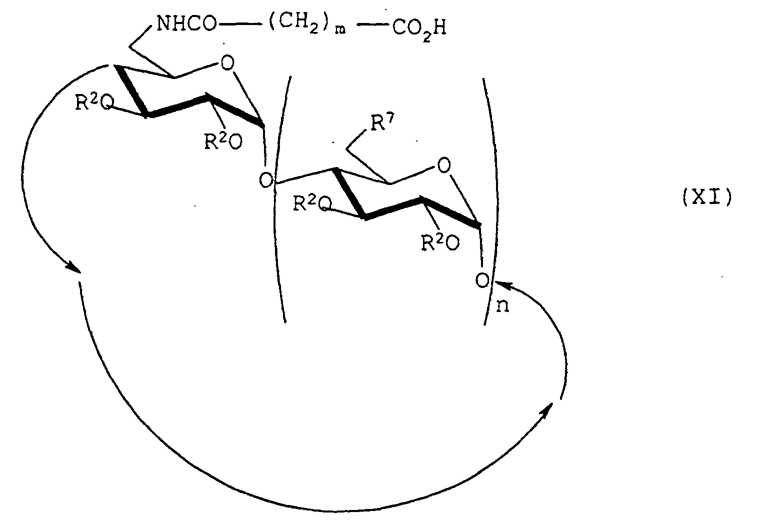 wherein all the R⁷ represent OR², or one of the R⁷ represents ―NHCO―(CH₂)ₘ―COOH and the other R⁷ represent OR², and R², m and n are as defined above, and
d) react the derivative according to formula (XI) with a compound according to the formula NH₂-R¹ to obtain the cyclodextrin derivative according to formula (I) defined above.

10. Inclusion complex of a cyclodextrin derivative according to any of claims 1 to 7, and a hydrophobic compound.

11. Complex according to claim 10, wherein the hydrophobic compound is chosen from steroids, neurotropes, bacteriostatics, vitamins, vascular wall tonics and contrast agents.

12. Complex according to claim 10, wherein the hydrophobic compound is chosen from 16-iodo-3-methylhexadecanoic acid, dothiepin, chloramphenicol, vitamin A and esculin.

13. Aqueous solution of nanoparticles of a cyclodextrin derivative according to any of claims 1 to 7 or an inclusion complex according to any of claims 10 to 12.

14. Organised surfactant system comprising a cyclodextrin derivative according to any of claims 1 to 7 or an inclusion complex according to any of claims 10 to 12.

15. System according to claim 14 wherein the surfactant is a phospholipid.

16. Aqueous solution comprising in solution a combined system formed from phospholipid or membrane protein vesicles, and at least one cyclodextrin derivative according to any of claims 1 to 7 or at least one cyclodextrin derivative inclusion complex according to any of claims 10 to 12.
